(19) Europäisches Patentamt
European Patent Office
Office européen des brevets

(11) **EP 3 671 217 A1**

(12) **EUROPÄISCHE PATENTANMELDUNG**

(43) Veröffentlichungstag:
**24.06.2020 Patentblatt 2020/26**

(51) Int Cl.:
*G01N 33/84* (2006.01)

(21) Anmeldenummer: 18214270.3

(22) Anmeldetag: **19.12.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Justus-Liebig-Universität Gießen**
**35390 Gießen (DE)**

(72) Erfinder:
• **Scheibe, Susan**
  **61169 Friedberg (DE)**
• **Mayer, Konstantin**
  **35423 Lich (DE)**
• **Weissmann, Norbert**
  **35415 Pohlheim (DE)**

(74) Vertreter: **Stumpf, Peter**
**c/o TransMIT GmbH**
**Kerkrader Strasse 3**
**35394 Gießen (DE)**

(54) **ERFINDUNG BETREFFEND DIAGNOSTIK EINER TRANSPLANTATABSTOSSUNG**

(57) Die vorliegende Erfindung betrifft ein *in vitro* Verfahren zum Nachweis von reaktiven Sauerstoff- und Stickstoffspezies (ROS/RNS) in Körperflüssigkeiten oder Gewebeproben am Beispiel der Lunge. Anhand der Messergebnisse kann potentiell innerhalb von Minuten eine Entscheidung darüber getroffen werden, ob eine akute Transplantatabstoßung wahrscheinlich ist.

Abbildung 1:

EP 3 671 217 A1

**Beschreibung**

Die vorliegende Erfindung betrifft ein *in vitro* Verfahren zur Diagnose einer akuten Transplantatabstoßung. Das Verfahren basiert im Kern auf dem direkten Nachweis von reaktiven Sauerstoff- (ROS) und Stickstoffspezies (RNS) in einer biologischen Probe z.B. von einer Körperflüssigkeit oder einer Gewebeprobe am Beispiel der Lunge. Anhand der Messergebnisse kann potentiell innerhalb von Minuten eine Entscheidung darüber getroffen werden, ob eine akute und höhergradige Transplantatabstoßung wahrscheinlich ist.

Die allogene Lungentransplantation stellt eine gut etablierte Therapieoption für Patienten mit chronisch irreversiblen Lungenerkrankungen der Atemwege im Endstadium dar. Das mittlere Langzeitüberleben liegt derzeit bei 5,6 Jahren. Eine der Hauptkomplikationen bei transplantierten Patienten ist eine akute Abstoßungsreaktion (*acute rejection,* AR). Von einer AR zu unterscheiden ist die heute *Chronic Lung Allograft Dysfunction* (CLAD) benannte chronische Verlaufsform, die u. a. als Folge des Gewebeuntergangs vieler aufeinanderfolgender AR-Ereignisse angesehen wird. Gemäß des von der *International Society for Heart and Lung Transplantation* (ISHLT) geführten Registers erleiden 50 bis 70% der Patienten mindestens eine akute Abstoßungsreaktion (AR) innerhalb des ersten Jahres nach der Transplantation. Die Häufigkeit und Schwere einer AR ist mit einem ansteigenden Risiko einer chronischen Dysfunktion des Lungentransplantats assoziiert. Die Erkennung und Behandlung einer frühzeitig erkannten AR könnte somit die Langzeitprognose des Transplantats verbessern.

Der derzeitige, auf transbronchiale Biopsien (TBB) gestützte Goldstandard ist ein sehr invasives Verfahren, das erhebliche Risiken für den Patienten birgt (z. B. Blutung, Pneumothorax, Infektion). Dieses Verfahren ist sowohl zeitaufwendig als auch fehleranfällig und liefert oft keine eindeutigen Ergebnisse. Nachteilig ist auch, dass es hinsichtlich der ordinalskalierten Abstoßungsgrade nach ISHLT-A (Lungenparenchym) und ISHLT-B (Bronchialgewebe) in seinen Ergebnissen subjektiv gefärbt ist, was sich statistisch in einer hohen Variabilität bei histopathologischer Bewertung desselben Materials durch verschiedene Untersucher zeigt.

Als Ergebnis einer Studie nach dem Stand der Technik (LARGO-Studie) zeigt Tab. 1 die Ergebnisse der Klassifizierung von Lungengewebeproben nach ISHLT-A ($A_0$... $A_4$) bzw. ISHLT-B ($B_0$... $B_4$), welche durch Pathologen der an der Studie teilnehmenden lokalen Behandlungszentren vorgenommen wurden. Diese werden hier in Form einer Kreuztabelle den Einstufungen der gleichen Gewebeproben durch die Pathologen des Consensus Panel der Studie gegenüber gestellt (nach Arcasoy, 2011).

Die eingekreisten Diagonalelemente repräsentieren die Frequenzen der übereinstimmend klassifizierten Befunde. Die Prozentsätze am Rand zeigen deren Anteil an allen Befunden der gleichen Zeile. Die große Zahl der als $A_x$ oder $B_x$ klassifizierten Fälle von nicht bewertbaren parenchymalen bzw. bronchialen Gewebeproben beruht auf der Einschätzung des Consensus Panels, dass diese Gewebeproben nicht den geforderten Qualitätsstandards entsprachen, wohingegen sie durch die lokalen Transplantationszentren als akzeptables Material klassifiziert worden waren. Insbesondere hinsichtlich der Einstufung als geringgradige parenchymale Abstoßung $A_1$ oder als geringgradige bronchiale Abstoßung $B_1$ zeigt sich eine sehr geringe Übereinstimmung und damit eine große Unsicherheit der histopathologischen Diagnostik, die sich nur über das nicht-Vorliegen einer Abstoßung ($A_0$ bzw. $B_0$) weitgehend einig ist. Während die Ergebnisse dieser Studie letztlich Mittelwerte repräsentieren, die sehr viel schlechter oder auch sehr viel besser als die Befundungsqualität an einzelnen lokalen Zentren sein können, so weist dies doch auf das grundsätzliche Problem hin, dass die lokale Qualität der Befundung bislang nicht überwacht wird. Anders als im klinischen Labor seit langem üblich, werden Ringversuche zur Qualitätssicherung der histopathologischen Diagnosen bislang nicht durchgeführt, was letztlich auch die Etablierung neuer diagnostischer Methoden behindert.

Ein wesentlicher Mangel der bisherigen Methode liegt auch darin, dass die auf Biopsiematerial gestützte Diagnose erst nach drei bis sechs Tagen vorliegt. Sie ist zudem in ihrer Aussagekraft nicht selten (in 7 % der hier untersuchten Fälle hinsichtlich ISHLT-A bzw. in 2 % hinsichtlich ISHLT-B) dadurch limitiert, dass zu wenig Material an parenchymalen bzw. bronchialen Gewebeproben für eine Beurteilung vorhanden oder geeignet war. Aufgrund der notwendigerweise beschränkten Zahl an Bioptaten kann die getroffene Auswahl die tatsächlich von einer AR betroffenen Lungenareale auch verfehlen.

In hochakuten Fällen bedeutet die Zeitverzögerung bei der Diagnose, dass für die dann dringliche Therapieentscheidung zur verstärkten Immunsuppression eher weiche klinische Kriterien wie der Abfall der Einsekundenkapazität ($FEV_1$) von > 10%, der Nachweis eines Pneumothorax, einer Bronchialstenose oder einer pulmonalen Kongestion mittels bildgebender Verfahren wie eine Röntgenaufnahme des Thorax oder eine thorakale Computertomographie Anwendung finden, welche jedoch weder als spezifisch noch als sensitiv angesehen werden.

Zusätzliche Methoden, die sich auf die Bestimmung potentieller Marker einer AR stützen sind erforderlich, um die Diagnosestellung zu erleichtern und letztendlich das Langzeitüberleben des Transplantats zu verbessern. Dazu zählt die zytologische Analyse der Leukozytensubpopulationen in der bronchoalveolären Lavage Flüssigkeit (BALF) und im Vollblut. Die Bedeutung eines veränderten Zellmusters für die Diagnose einer AR wird kontrovers diskutiert, da verschiedene Studien teils widersprüchliche Ergebnisse zeigen.

Eine BALF-Neutrophilie ($\geq$12% der Leukozyten) bei gleichzeitigem Ausschluss einer Infektion erhöht den Verdacht auf eine AR. Eine hohe Anzahl an Lymphozyten in der BALF ist mit einer AR assoziiert. Verschieden Studien wiesen bei einer Lymphozytose (>20 %) eine akzeptable Spezifität für eine AR nach; die Sensitivität hingegen war meist gering. Eosinophile sind bei stabilen Patienten selten zu finden; werden sie dennoch nachgewiesen ist die Wahrscheinlichkeit des Vorliegens einer AR ebenfalls erhöht. Unklar ist die Bedeutung der Makrophagen in der BALF. In zahlreichen Studien war der Anteil an Makrophagen, nicht jedoch deren Gesamtzahl bei Vorliegen einer AR vermindert.

Reaktive Sauerstoffspezies (*reactive oxygen species, ROS*) umfassen Sauerstoffradikale sowie hochreaktive Sauerstoffverbindungen. Reaktive Stickstoffspezies (*reactive nitrogen species, RNS*) umfassen hoch reaktive Stickstoffverbindungen und Stickstoffradikale. ROS und RNS werden von nahezu allen eukaryotischen Zellen produziert. Zu den wichtigsten biologisch relevanten ROS zählen u.a. Superoxid (auch Superoxid Anion, $O_2^{\bullet-}$), Hydrogenperoxid ($H_2O_2$), das Hydroxylradikal ($^{\bullet}OH$) und Lipidperoxid (LOOH). Zu den RNS gehört Stickstoffmonoxid ($^{\bullet}O$), Nitrit ($NO_2^-$) und Peroxinitrit ($ONOO^-$), das in der Reaktion von $O_2^{\bullet-}$ mit $^{\bullet}NO$ entsteht. In physiologischen Konzentrationen sind ROS/RNS essentiell für die Regulation von zellulären und systemischen Signalkaskaden. Sie werden außerdem von Zellen des angeborenen und adaptiven Immunsystems, vor allem von neutrophilen Granulozyten (Neutrophile) und Makrophagen zur Abtötung von Pathogenen eingesetzt. Ein exzessiver Anstieg der ROS/RNS-Konzentration kann zu oxidativen Schädigungen von Lipiden, Proteinen und Nukleinsäuren führen. ROS werden mit einer Beschleunigung von Alterungsprozessen, altersbedingten Behinderungen sowie der Pathogenese und Progression einer Vielzahl von Erkrankungen assoziiert. Es konnte nachgewiesen werden, dass sie allgemein bei entzündlichen Erkrankungen, daneben auch in der Pathogenese von neurodegenerativen, kardiovaskulären und pulmonalen Erkrankungen sowie der Tumorgenese involviert sind. Normalerweise reguliert ein fein abgestimmtes antioxidatives System die ROS/RNS Konzentration, um die zelluläre Redox-Homöostase (Gleichgewicht zwischen Oxidation und Reduktion) aufrechtzuerhalten.

Je nach Analysetechnik lassen sich einzelne Analyten der ROS/RNS nicht unabhängig voneinander quantifizieren, nachdem es bisher keine direkten Nachweismethoden gibt, was vor allem durch ihre Halbwertszeit im Bereich von Nanosekunden bis wenigen Sekunden bedingt ist.

## Aufgabe

Aufgabe der vorliegenden Erfindung ist ein verbessertes Verfahren zur einfachen, zeitnahen und zuverlässigen Diagnose einer Transplantatabstoßung in einer biologischen Probe.

## Lösung der Aufgabe

Diese Aufgabe wird erfindungsgemäß gelöst durch die Merkmale des Anspruchs 1. Vorteilhafte Ausgestaltungen des erfindungsgemäßen Verfahrens sind den Unteransprüchen zu entnehmen.

Es werden eigene wissenschaftliche Arbeiten durchgeführt, um die Eignung von ROS/RNS als Biomarker einer AR zu erforschen. Darin kann gezeigt werden, dass das Superoxid-Signal bzw. das Gesamtsignal aus Superoxid und Peroxinitrit ideale Biomarker für die Diagnose einer AR der Lunge sind. Ein direkter ROS/RNS-Nachweis als potentieller Biomarker einer AR ist bislang nicht bekannt. Das erfindungsgemäße Verfahren basiert auf einem Nachweis von ROS bzw. RNS in einer biologischen Probe eines Patienten, sodass mit hoher Sensitivität und Spezifität erkennbar wird, ob eine akute Transplantatabstoßung vorliegt. Anhand dieser Diagnose wird dann eine adäquate Therapiewahl möglich.

Exemplarisch wird das Vorgehen anhand einer AR der Lunge beschrieben, wobei als biologische Probe primär BALF, aber auch eine Gewebeprobe, eine Körperflüssigkeit oder, zwecks Nachweis von $^{\bullet}NO$ bzw. $H_2O_2$, eine Atemgasprobe eingesetzt werden könnte. Die Ermittlung der Messergebnisse erfolgt rasch, sodass potentiell innerhalb von Minuten eine Entscheidung darüber getroffen werden kann, ob eine AR vorliegt.

Die Erfindung weist folgende Vorteile auf:

- Zeitnahe objektive Diagnose einer AR der Lunge innerhalb von Minuten bis maximal wenige Stunden
- Leicht verfügbares Probenmaterial (gasförmige, flüssige oder feste bzw. gefrorene biologische Probe)
- Nicht-invasives Verfahren bei Nutzung von Restmaterial der zytologischen Routinediagnostik
- Messung von ROS und/oder RNS
- Anwendung des Verfahrens auch zur Diagnostik einer AR anderer Gewebe und Organe (wie Herz, Leber, Niere, Darm, Gliedmaßen, Zellen bzw. Zellverbände)
- Der Gegenstand der Erfindung kann Teil eines kompakten Analysesystems werden, welches Probenmaterial automatisch entgegennimmt, geeignet aufbereitet und spezifisch für das jeweilige Nachweisverfahren [Elektronen-Spin-Resonanz-Spektroskopie (ESR), Fluorometrie, Gaschromatographie (GC), HPLC, Lumineszenz, Redox-Sensoren usw.] auswertet

**Beschreibung**

Das erfindungsgemäße Verfahren zur Bestimmung einer akuten Transplantatabstoßung in einer biologischen Probe eines Patienten wird mit folgenden Schritten durchgeführt werden:

i) Messung der reaktiven Sauerstoffspezies (ROS) und/oder der reaktiven Stickstoffspezies (RNS) in der biologischen Probe, wobei der Analyt der Messung Superoxid ($O_2^{\bullet-}$) und/oder Peroxinitrit ($ONOO^-$) ist

ii) Vergleich des Messwerts der reaktiven Spezies aus Schritt i) mit dem zugehörigen Schwellwert bei einer zu wählenden Sensitivität oder Spezifität

iii) Entscheidung für das Vorliegen einer akuten Transplantatabstoßung, wenn der Messwert über dem zugehörigen Schwellwert liegt

In einer weiteren Ausführungsform werden in Schritt i) des erfindungsgemäßen Verfahrens zusätzlich weitere radikalische bzw. hochreaktive Verbindungen wie $^\bullet OH$, $LOOH$, $^\bullet NO$, $ONOO^-$, $NO_2^-$ oder $H_2O_2$, aber auch die radikalischen Formen der Ascorbinsäure ($Asc^\bullet$) und des $\alpha$-Tocopherols ($Toc^\bullet$) gemessen.

Die ROS/RNS Messwerte werden mittels Elektronen-Spin-Resonanz-Spektroskopie gemessen. Alternativ erfolgt die Messung aus Schritt i) mittels Fluoreszenz- oder Lumineszenz-basierten Verfahren, mittels Redox-Sensoren oder mittels HPLC, GC/MS, LC/MS oder der spektrophotometrischen Messung der Cytochrom-c Reduktion.

Die biologische Probe ist eine gasförmige, flüssige oder feste Probe, wobei eine flüssige Probe Gallenflüssigkeit, Pankreassekret, Liquor cerebrospinalis, Tränenflüssigkeit, Gelenkflüssigkeit, Ascites, Blut, Lymphflüssigkeit oder Urin umfasst oder eine durch Spülung verdünnte Körperflüssigkeit wie ELF, Pleuraflüssigkeit, Perikardflüssigkeit, Peritonealflüssigkeit ist oder eine verflüssigte feste Probe ist. Eine feste Probe umfasst ein Bioptat oder eine eingefrorene flüssige Probe. Eine gasförmige Probe umfasst Atemluft eines Patienten oder eine durch Katheter aus Körperhöhlen entnommene gasförmige Probe.

In einer weiteren Ausführungsform des erfindungsgemäßen Verfahrens wird nach Schritt iii) ein zusätzlicher Schritt iv) durchgeführt, in dem die Makrophagenkonzentration in der biologischen Probe ermittelt wird.

In einer weiteren Ausführungsform des erfindungsgemäßen Verfahrens wird nach Schritt i) ein zusätzlicher Schritt v) durchgeführt wird, in dem ein Flüssigkeitsvolumen als Recovered [%]-Wert ermittelt wird, wobei die Flüssigkeit über eine BAL gewonnen wird und wobei das instillierte als auch das nach Absaugen zurückgewonnene Volumen (BALF) ermittelt wird.

In einer weiteren Ausführungsform des erfindungsgemäßen Verfahrens wird in Schritt iv) zusätzlich auch die Eosinophilenkonzentration in der biologischen Probe ermittelt und ein Score-Wert nach Gleichung 2 ermittelt:

$$\text{Score} = \text{Superoxid} / \text{Makrophagen} \times (\text{Eosinophile} + 1) / \text{Recovered}$$

In einer weiteren Ausführungsform unterscheiden sich die Schwellwerte je nach Geschlecht des Patienten.

Das erfindungsgemäße Verfahren wird zur Therapieüberwachung und/oder Dosisanpassung von Immunsuppressiva zur Therapie der akuten Transplantatabstoßung verwendet. Das erfindungsgemäße Verfahren wird zur Qualitätssicherung histopathologischer Befunde verwendet.

Gegenstand der Erfindung ist auch eine Vorrichtung, zur Durchführung des erfinderischen Verfahrens, wobei die Vorrichtung umfasst

- eine Messeinheit zur Eingabe der biologischen Probe und zur Messung von ROS und/oder RNS in der biologischen Probe und
- eine Auswerteinheit, die mit der Messeinheit verbunden ist, in der die Messwerte aus der Messeinheit mit jeweils zugehörigen Schwellwerten abgeglichen werden und die Auswertung erfolgt, ob eine akute Transplantatabstoßung vorliegt oder nicht.

Die Messeinheit und die Auswerteinheit wirken automatisiert zusammen und die Auswerteinheit umfasst alternativ eine Einheit des maschinellen Lernens (KI).

**Biologische Probe**

Die im Verfahren verwendete biologische Probe ist eine Probe eines Menschen oder alternativ eines Säugetieres wie z.B. Maus, Hund, Katze, Pferd, Affe, Schwein. Üblicherweise werden diese Spezies transplantiert und dienen als Empfänger von Transplantaten. Die biologische Probe ist eine gasförmige, flüssige oder feste (bzw. gefrorene) Probe, wobei die flüssige Probe eine ggf. durch Spülung verdünnte Körperflüssigkeit wie die *Epithelial Lining Fluid* (ELF, eine die Alveolen bronchialseitig benetzende, proteinreiche Flüssigkeit) Pleuraflüssigkeit, Perikardflüssigkeit, Peritonealflüssigkeit, Gallenflüssigkeit, Pankreassekret, Blut, Liquor cerebrospinalis, Lymphflüssigkeit, Gelenkflüssigkeit, Tränenflüssigkeit, Ascites oder Urin sein kann, welche über entsprechend platzierte Katheter teilsweise auch gezielt aus dem Abstrom des jeweils transplantierten Organs entnommen werden. Gasförmige Proben können aus Körperhöhlen oder der Ausatemluft entnommen werden. Bevorzugt ist die biologische Probe eine Flüssigkeit, wie sie z. B. über eine bronchoalveoläre Lavage (BAL) gewonnen wird. Die BAL ist ein medizinisches Verfahren, bei dem ein flexibles Bronchoskop in das Bronchialsystem der Lunge eingeführt wird, wobei eine Spülflüssigkeit instilliert und dann zur Untersuchung wieder abgesaugt wird. Dieses Verfahren wird typischerweise zur Diagnose von Lungenerkrankungen an einem Patienten durchgeführt und auch bei lungentransplantierten (Ltx)-Patienten angewendet. Bei dieser Gelegenheit werden durch Biopsie normalerweise auch feste Proben des Lungengewebes entnommen.

Die feste Probe ist ein Bioptat, das homogenisiert, verflüssigt oder anderweitig aufgetrennt wird. Die feste Probe ist ein Teil eines Organs wie z. B. Lunge, Leber, Niere, Herz, Darm, Pankreas, Gliedmaßen, Knochen, Knorpel, gemeinhin Teil eines Gewebeverbands oder auch nur einzelne Zellen. Eine feste Probe kann natürlich auch eine gefrorene, vormals flüssige Probe sein.

**Schritt i) Messung der reaktiven Sauerstoffspezies (ROS) und der reaktiven Stickstoffspezies (RNS)**

Aufgrund der hohen Reaktivität und der kurzen Halbwertszeit von ROS und RNS im Bereich von Nanosekunden bis Sekunden sowie zahlreicher zellulärer Mechanismen, diese zu entfernen, sind Sonden erforderlich, die schnell mit reaktiven Spezies reagieren, u. a. um mit endogenen Antioxidantien konkurrieren zu können. Die Messung von ROS erfolgt bevorzugt durch Erfassung von $O_2^{\bullet-}$ mittels ESR (Elektronen-Spin-Resonanz-Spektroskopie) durch Einsatz von Hydroxylamin-Spin *probes,* wie etwa die hier verwendete Verbindung *3-methoxycarbonyl-2,2,5,5-tetramethyl-pyrrolidine -1-oxyl* (CMH). CMH reagiert mit hoher Reaktionsgeschwindigkeit ($ko_2^{\bullet}- 0{,}84 \times 10^3$ bis $1{,}2 \times 10^4$ $M^{-1}s^{-1}$) hauptsächlich mit $O_2^{\bullet-}$; kann jedoch auch durch das zu den RNS gehörende $ONOO^-$ oxidiert werden. In dieser Reaktion entsteht das über Stunden stabile CM-Radikal ($CM^{\bullet}$), welches per ESR quantifiziert werden kann. Um die $O_2^{\bullet-}$-Produktion unabhängig von der in der Probe enthaltenen $ONOO^-$-Konzentration zu quantifizieren, wurden zellhaltige Parallelansätze sowohl mit CMH allein als auch zusätzlich gemeinsam mit einer pegylierten und deshalb membrangängigen Superoxid-Dismutase (pSOD) inkubiert. Die Differenz der ESR-Signale von zwei zellhaltigen Parallelansätzen, von denen einer mit CMH alleine und ein zweiter außer mit CMH auch mit pSOD inkubiert wurde, liefert ein Maß für die $O_2^{\bullet-}$-Konzentration in der Probe. Die Differenz aus dem ESR-Signal der mit allein mit CMH inkubierten, zellhaltigen Proben und dem Signal aus zellfreien, aber ebenfalls mit CMH versetzten Kontrollproben (Hintergrundsignal) kann als Maß für die Summe aus $O_2^{\bullet-}$ und $ONOO^-$ verwendet werden.

ESR-Proben können sowohl im flüssigen als auch im festen (bzw. tiefgefrorenen) Zustand untersucht werden, wobei aus praktischen Gründen Letzterem oft der Vorzug gegeben wird. Radikalische Analyten mit langer Halbwertszeit wie etwa das Ascorbyl-Radikal ($Asc^{\bullet}$) können auch ohne den Umweg über eine *Spin probe* gemessen werden. Dies gilt wenigstens prinzipiell auch für gasförmige Analyten wie das im Atemgas nachweisbare $^{\bullet}NO$, dessen Halbwertszeit mit 10-30 Sekunden angegeben wird.

**Alternative Methoden zur Messung der reaktiven Sauerstoffspezies (ROS) und der reaktiven Stickstoffspezies (RNS) in Schritt i)**

Alternativ kann die Bestimmung der ROS-Konzentration durch Fluoreszenz-basierte (u.a. DHE, DCFDA, RoGFP, HyPer, MitoSOX) oder Lumineszenz-basierte (u.a. Luminol, Lucigenin) Methoden erfolgen. Außerdem werden Methoden zum ROS-Nachweis eingesetzt, die sich auf HPLC, LC/MS oder der spektrophotometrischen Messung der Cytochrom-c Reduktion stützen. $^{\bullet}NO$ kann auch mit $^{\bullet}NO$-sensitiver Elektroden in Echtzeit in der Gasphase gemessen werden. Die Konzentration von $ONOO^-$ kann u.a. durch Messung von Nitrotyrosin mittels HPLC oder mittels immunohistochemischer Methoden bestimmt werden. Obwohl sehr wünschenswert, ist bisher keine Methode bekannt, die alle ROS oder alle RNS in ihren Anteilen bzw. in ihrer Gesamtheit erfassen könnte.

**Schritt ii) Vergleich des Messwerts der reaktiven Spezies aus Schritt i) mit einem zugehörigen Schwellwert ("Threshold") und Ermittlung ob eine AR vorliegt**

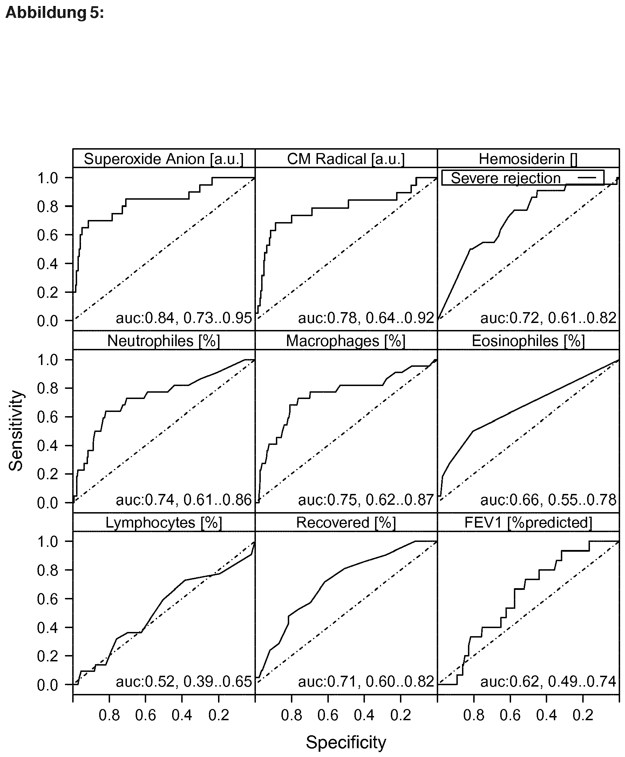

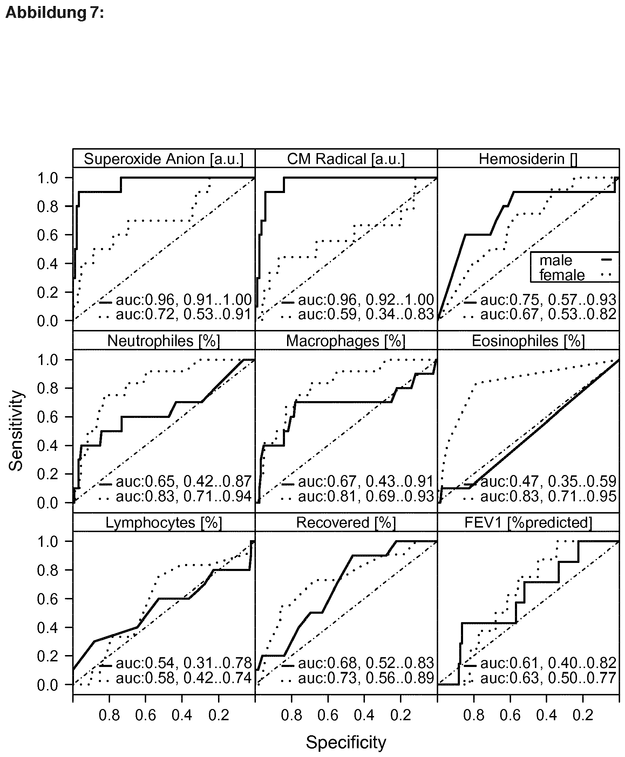

Für das beschriebene, ESR-gestützte Protokoll werden in Abhängigkeit von Sensitivität und Spezifität Schwellwerte ("Thresholds") für $O_2^{\bullet-}$ und das CM-Radikal in Bezug auf die Diagnose einer schweren AR in den Tab. 3, 4, 5 und 6b in 5 % Schritten der Spezifität aufgeführt, welche durch einen *Receiver Operating Characteristic* (ROC)-Algorithmus auf Basis der Studiendaten berechnet wurden. In vollständiger Form liegen diese Datensätze auch hinter den ersten beiden ROC-Diagrammen in Abb. 5 und 7. Eine "schwere AR" ist nach Gleichung 1 so definiert, dass die zugrundelie-

genden biologischen Proben als $\geq A_2$ bei beliebiger B-Gradierung (inklusive $B_x$) oder $\geq B_2$ bei beliebiger A-Gradierung (inklusive $A_x$) eingestuft wird.

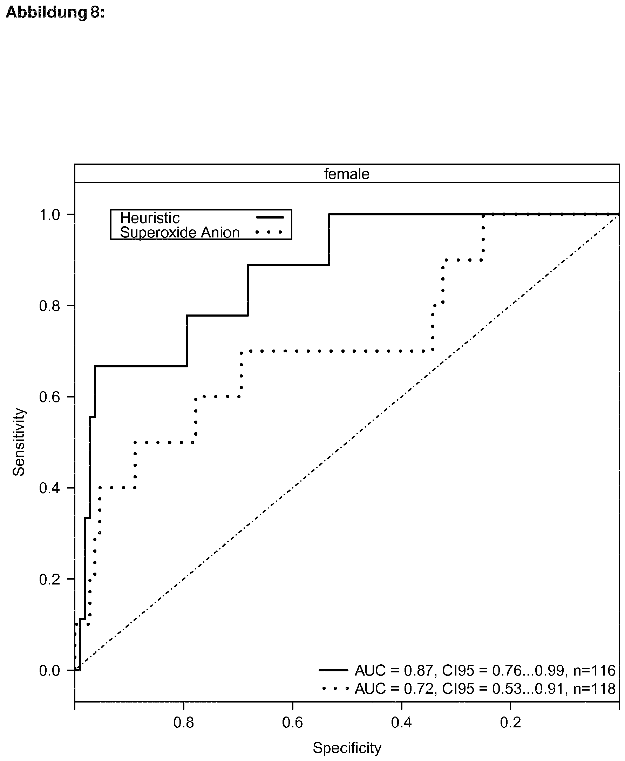

Abb. 7 zeigt, dass diese Schwellwerte für Superoxid und CM-Radikal in Bezug auf männliche und weibliche Patienten signifikant verschieden sind (95 % Konfidenzintervalle überlappen sich nicht). Bei männlichen Patienten wird im klinisch interessanten Bereich hoher Spezifität auch eine sehr hohe Sensitivität erreicht, wobei die Messung des CM-Radikals (als Maß für die Summe aus $O_2^{\bullet-}$ und $ONOO^-$) fast ebenso gute Ergebnisse erzielt wie diejenige des Superoxids. Dies kann bei einer ESR-Messung von Vorteil sein, weil damit ein Ansatz (parallele Inkubation mit pSOD) entfällt. Bei Frauen, deren Immunsystem auch hinsichtlich der anderen untersuchten Parameter unterschiedlich zum männlichen reagiert, erbringt die Berechnung eines Scores nach Gleichung 2 und dessen Abgleich mit den zugehörigen, in Tab. 6a dokumentierten Schwellwerten etwas bessere Ergebnisse (Abb. 8) als die direkte Verwendung der Superoxid-Konzentrationen mit den Schwellwerten der Tab. 7. Ähnlich erbringt die Verwendung der für Männer spezifischen Superoxid-Schwellwerte in Tab. 6b ausweislich Abb. 7 sehr viel bessere Ergebnisse als die Verwendung der Schwellwerte in Tab. 3 (Superoxid) oder Tab. 4 (CM-Radikal), welche das Geschlecht nicht berücksichtigen.

**Kombination der Messwerte aus Schritt ii) mit den Messwerten weiterer Biomarker**

Alternativ werden die Messwerte aus Schritt ii) mit den Messwerten weiterer Biomarker zu einem Vorhersagemodell verbunden. Weitere Biomarker sind z. B. das Geschlecht des Patienten, von dem die biologische Probe gewonnen wurde, ggf. auch das Geschlecht des Organspenders, die Verteilung der Leukozytensubpopulationen (Neutrophile, Makrophagen, Eosinophile und Lymphozyten) als auch, insbesondere bei Neutrophilen oder Makrophagen, deren Aktivierungsstatus oder Immunphänotyp. Zusätzlich kann der Wert des Hämosiderin-Scores, der Anteil an zurückgewonnener BALF, Zytokinkonzentrationen, die Konzentration rezyklierbarer exogener (z. B. $\alpha$-Tocopherol, Ascorbat) als auch endogener Bestandteile des antioxidativen Systems wie Glutathion (GSH/GSSG Verhältnis), Glutathionperoxidase, SOD und Katalase, die Konzentrationen von Zerfallsprodukten der DNA-, Protein- und Lipidschädigung oder auch Hitzeschockproteine als Ausdruck von Reparaturprozessen der durch oxidativen Stress geschädigten Proteine, oder die Konzentration von Gasen wie $^{\bullet}NO$ gemessen und in das Vorhersagemodell eingebunden werden. Insbesondere im Hinblick auf die Validität bzw. Normierung solcher Messungen ist auch die Verdünnung der ELF in der zurückgewonnenen BALF von Interesse, die z. B. über die parallele Messung der Harnstoffkonzentrationen im Blutplasma und in der BALF ermittelt werden kann. Daneben können auch Ergebnisse der klinischen Labor- und Funktionsdiagnostik einbezogen werden. Abhängig von der Art des transplantierten Organs könnte z. B. die Kreatininclearance der Niere, die Einsekundenkapazität der Lunge ($FEV_1$) oder die Ejektionsfraktion des Herzens zur Maximierung der diagnostischen Sensitivität bzw. Spezifität beitragen.

**Verwendung des erfindungsgemäßen Verfahrens**

Das erfindungsgemäße Verfahren kann verwendet werden, um anhand einer biologischen Probe festzustellen, ob eine AR vorliegt. Zusätzlich kann das erfindungsgemäße Verfahren zur Therapie- und Verlaufskontrolle eingesetzt werden. Folglich kann das erfindungsgemäße Verfahren auch zur Dosisanpassung von zur Unterdrückung einer AR eingesetzten Immunsuppressiva eingesetzt werden, indem über einen zeitlichen Verlauf mehrere biologische Proben gemessen und ausgewertet werden. Die Entscheidung zur individuellen Dosiserhöhung oder -absenkung kann damit anhand des zeitlichen Verlaufs der ROS- bzw. RNS-Konzentration getroffen werden. Zusätzlich kann das erfindungsgemäße Verfahren im Rahmen der Qualitätssicherung histopathologischer Befunde verwendet werden: Als Anstoß zur Neubegutachtung oder Neuerhebung bei hinsichtlich des Abstoßungsgrades divergenten Befunden und letztlich als Anstoß zur Fortentwicklung des Begutachtungsstandards und der hierfür eingesetzten Labortechnik.

**Vorrichtung zur Durchführung des erfinderischen Verfahrens**

Die Vorrichtung umfasst

- eine Messeinheit zur Eingabe der biologischen Probe und zur Messung von ROS und/oder RNS in der biologischen Probe und
- eine Auswerteinheit, die mit der Messeinheit verbunden ist, in der die Messwerte aus der Messeinheit mit jeweils zugehörigen Schwellwerten ("Thresholds") abgeglichen werden und die Auswertung erfolgt, ob eine akute Transplantatabstoßung vorliegt oder nicht.

Die Messeinheit und die Auswerteinheit wirken automatisiert zusammen und die Auswerteinheit umfasst alternativ eine Einheit des maschinellen Lernens (KI). Allen Methoden gemeinsam ist eine Vorrichtung zur Entgegennahme von Probenmaterial, zur Aufbereitung und zur Messung, die prinzipiell in einem kompakten Gerät vereinigt werden können, wobei auch parallele Aufbereitungs- und Analysekanäle (z.B. ESR, Gaschromatographie, Differentialzytologie, Zytokinbestimmung usw.) denkbar sind. Ein solches Gerät könnte auch eine fortschreitend über Rechnerschnittstellen aktualisierbare Datenbank, welche zurückliegende Fälle nebst deren histopathologischen Klassifizierungen, klinische Labordaten, Funktionsdaten, ärztliche Beurteilungen sowie *ex post* Informationen (z.B. Sektionsdiagnosen) über den Gesamtverlauf enthält, auf deren Grundlage ein Modell des maschinellen Lernens die Einstufung der Transplantatabstoßung vornehmen kann. Eine fortschreitende Aktualisierbarkeit der Datenbasis ist für eine maximale Vorhersagegüte schon wegen der kontinuierlich anwachsenden Fallzahl von Vorteil. Sie ist aber auch deshalb erstrebenswert, weil sich das lokale Patientenkollektiv hinsichtlich Vor- und Begleiterkrankungen, Behandlungsmethoden, Genetik, Umweltfaktoren, Alter, zeitlicher Abstand zur Transplantation usw. zwischen den einzelnen Behandlungszentren unterscheidet. Dies wiederum beeinflusst die *a priori* Wahrscheinlichkeiten für eine bestimmte Diagnose, weshalb bekanntlich jedes größere Behandlungszentrum mit einem eigenen klinischen Labor lokale Referenzbereiche vorhält und pflegt.

**Ausführungsbeispiele**

**Probenvorbereitung für Elektronen-Spin-Resonanz-Spektroskopie (ESR)**

Als biologische Probe im Kontext einer AR der Lunge wird beispielsweise die über die BAL-Prozedur zurückgewonnene Spülflüssigkeit verwendet. Üblicherweise werden maximal 150 ml sterile, physiologische Kochsalzlösung zum Spülen der Lunge eingesetzt. Das instillierte als auch das nach Absaugen zurückgewonnene Volumen wird dokumentiert und der Anteil des zurückgewonnenen am instillierten Volumen berechnet ("Recovered [%]"). Die zurückgewonnene BALF wird nach Erhalt aus der Bronchoskopie zentrifugiert. hier bei 20° für 8 min bei 350 g. Anschließend wird das vornehmlich aus Leukozyten bestehende Zellpellet in geeignetem Medium z.B. in RPMI-1640 Medium aufgenommen. Die Zellen darin werden ein weiteres Mal abzentrifugiert, hier für 5 min bei 338 g. Im Anschluss wird das Medium abgenommen und verworfen. Das Zellpellet wird in einem geeignetem Puffer, z.B. Krebs-Henseleit-Puffer (KHB) resuspendiert. Die Zellzahl wird mittels einer Neubauer-Zählkammer bestimmt. Bei zellhaltigen Proben wird eine Zellkonzentration von $1 \times 10^6$ / ml eingesetzt. Die zellhaltigen Proben, als auch die zellfreien Kontrollen, welche bisher nur KHB enthalten, werden nach Zugabe von 50 U/ ml pSOD zunächst bei 37°C für 90 min inkubiert. Parallelansätze werden anstatt mit pSOD mit entsprechend zusätzlichen Volumen KHB unter den gleichen Bedingungen inkubiert. Anschließend wird die *Spin probe* CMH, resultierend in einer Endkonzentration von 0,5mM, zugefügt und für weitere 30 min bei 37°C inkubiert. Alle Ansätze enthalten letztlich ein Volumen von jeweils 300 $\mu$l. CMH reagiert mit dem hier vornehmlich durch Leukozyten produzierten $O_2^{\bullet-}$ und $ONOO^-$ und bildet als Reaktionsprodukt das stabile Radikal $CM^\bullet$, welches mittels ESR nachweisbar ist. Die ESR-Proben werden direkt nach Beendigung der Inkubationszeiten in 1 ml Spritzen (B. Braun, Melsungen) gefüllt, in Flüssigstickstoff schockgefroren und bis zur Messung bei -80°C gelagert. Ein zwischenzeitliches Einfrieren ist jedoch nicht erforderlich. Die Proben können bei Einhaltung immer gleicher Bedingungen hinsichtlich Temperatur, Lagerungs- und Messzeiten auch direkt im flüssigen Zustand gemessen werden.

**Parametereinstellungen zur ESR-Messung**

Die ESR-Messungen werden mit einem geeigneten Spektrometer wie z.B. dem EMXmicro Spektrometer im X-Band (ca. 9.4GHz) imit dem Resonator ER4119HS bei Raumtemperatur (20-22 °C) und eingeschalteter Klimaanlage durchgeführt. Die eingefrorenen Proben werden in ein geeignetes Gefäß, z.B. in einen ESR-Probenhalter aus doppelwandigem Quarzglas (Dewar), transferiert. Dieser wird vor jeder Probenmessung mit flüssigem $N_2$ gekühlt. Zur Vermeidung der Kondensation von Luftfeuchtigkeit am auch außen kalten Dewar wird der Resonator zusätzlich mit Stickstoff begast. Alternativ zum Dewar kann ein durch einen $N_2$-Thermostaten gekühlter Probenhalter verwendet werden. Am Gerät werden folgende, zur Messung gefrorener, zylindrischer Proben mit einem Durchmesser von 4,5 mm und einer Höhe von 19 mm (300 $\mu$l) geeignete Parameter eingestellt: g-Factor 2,0063, Center Field 3353 bis 3367 Gauß, Microwave Power 3 mW, Receiver Gain 50dB, Time Constant 10,24 ms, Number of Averages 6, Sweep Time 10s, Sweep Width 200 G, XPoints 2000, Modulation Amplitude 2G, Modulation Frequency 100kHz. Das Ergebnis der Messungen sind Intensitätswerte in willkürlichen Einheiten (a.u., *arbitratry units*), die bei immer gleicher Probengeometrie, Temperatur und Parametereinstellungen untereinander vergleichbar und prinzipiell auch in Einheiten der Konzentration ($\mu$mol/l) kalibrierbar sind.

**Reklassifizierung der histopathologischen Diagnosen**

Um die in Tab. 1 dargestellten Unschärfen insbesondere hinsichtlich $A_1$ und $B_1$ zu kompensieren (sehr geringe Übereinstimmung), als auch um dem als Folge einer fachgerechten immunsuppressiven Therapie niedrigen Anteil an untersuchten Patienten Rechnung zu tragen, bei denen überhaupt eine Abstoßung auftritt (linksschiefe Verteilung: *a priori* kann mit kleiner Irrtumswahrscheinlichkeit die Diagnose "keine Abstoßung" angenommen werden), wurden die beiden für jeden Patienten parallel vorliegenden Diagnosen nach ISHLT-A und ISHLT-B gemäß Gleichung 1 in die drei Gruppen "keine", "moderate" und "schwere" Abstoßung reklassifiziert.

**Ergebnisse**

**Beispiel 1:** Analyse der $O_2^{\bullet-}$- und $CM^{\bullet}$-Konzentration und der BALF-Charakteristika nach ISHLT-A (1996+2006) und ISHLT-B (1996) und klinischer Routinedaten. Es zeichnet sich ein positiver Trend zwischen $O_2^{\bullet-}$- und $CM^{\bullet}$-Konzentration und Neutrophilen mit zunehmenden Abstoßungsgrad ab. Eosinophile konnten in der BALF nur selten (in ca. 10% der Fälle) nachgewiesen werden, jedoch wiesen 20 von 22 Patienten mit einer schweren AR diese Population in der BALF auf. Ein negativer Trend hinsichtlich der Schwere einer AR ist bei Makrophagen, Recovered, der $FEV_1$ und dem Hämosiderin-Score erkennbar. Bei den Lymphozyten zeigte sich kaum ein Unterschied.

**Beispiel 2:** $O_2^{\bullet-}$- und $CM^{\bullet}$-Konzentrationen sowie anderer BAL-Charakteristika. Die in Bsp. 1 sehr differenzierte Klassifizierung der Abstoßungsgrade nach ISHLT-A und ISHLT-B wurde gemäß Gleichung 1 zusammengefasst. Die Beschreibung der Ergebnisse ist analog zu Beispiel 1.

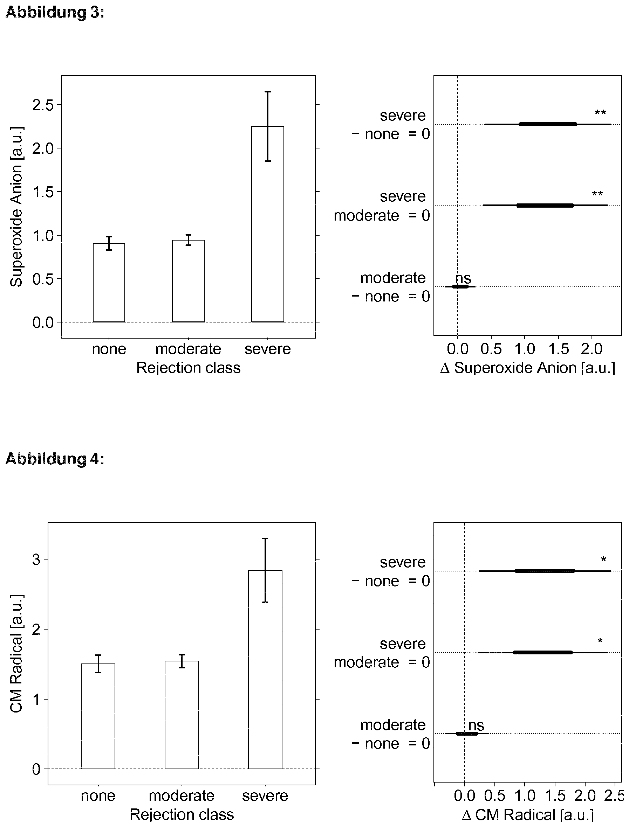

**Beispiel 3:** Erhöhte $O_2^{\bullet-}$-Konzentration bei einer schweren AR. Die nach Gleichung 1 negativen Fälle lassen sich von moderaten bisher nicht unterscheiden (Abb. 3, links), was in der bereits beschriebenen Schwierigkeit der histopathologischen Unterscheidung der $A_0$ von $A_1$ bzw. $B_0$ von $B_1$ - Diagnosen begründet liegen kann. Dagegen ist die $O_2^{\bullet-}$-Konzentration bei Vorliegen einer schweren AR mit ca. 2,2 a.u. gegenüber den $O_2^{\bullet-}$-Konzentrationen bei nicht-Bestehen oder bei Vorliegen einer moderaten AR (0,9 a.u.) um etwa 1,3 a.u. hochsignifikant erhöht (Effektplot, Abb.3, rechts). Die Berechnung erfolgte mittels eines Gamma-verteilten glm-Modells mit ausweislich des (hier nicht dargestellten) QQ-Plots unauffälligen Residuen auf Basis der in Tab. 2 aufgeführten Fallzahlen.

**Beispiel 4:** Erhöhte $CM^{\bullet}$-Konzentration bei einer schweren AR. Die nach Gleichung 1 negativen Fälle lassen sich von moderaten ($A_1$ oder $B_1$) bisher nicht unterscheiden (Abb. 4, links), was in der bereits beschriebenen Schwierigkeit der histopathologischen Unterscheidung der $A_0/A_1$ bzw. $B_0/B_1$ Diagnosen begründet liegen kann. Dagegen ist die $CM^{\bullet}$-Konzentration bei Vorliegen einer schweren AR mit ca. 3,0 a.u. gegenüber dem Nichtbestehen einer AR sehr signifikant bzw. bei Vorliegen einer moderaten AR hochsignifikant erhöht (Effektplot, Abb.4, rechts). Die Berechnung erfolgte mittels eines Gamma-verteilten glm-Modells mit ausweislich des (hier nicht dargestellten) QQ-Plots unauffälligen Residuen auf Basis der in Tab. 2 aufgeführten Fallzahlen.

**Beispiel 5:** Unter Verwendung des R-Pakets *pROC* wurden die Ergebnisse der $CM^{\bullet}$- und $O_2^{\bullet-}$-Bestimmungen, des BALF-Zellprofils und einiger weiterer klinischer Parameter (u.a. $FEV_1$, Recovered [%]) mittels ROC-Kurven auf deren Vorhersagegüte hinsichtlich des Vorliegens einer schweren AR untersucht. Weil in die Berechnung von ROC-Kurven die Prävalenz (hier also der Anteil an allen untersuchten Ltx-Patienten, die zum Zeitpunkt der bioptischen Diagnostik tatsächlich an einer schweren AR litten) grundsätzlich nicht mit eingeht, kann die klinische Relevanz direkt aus dem Verlauf der Kurven abgeschätzt werden (Abb. 5). Die höchste Vorhersagegüte zeigt die $O_2^{\bullet-}$-Konzentration mit einer AUC von 0,84.

**Beispiel 6:** Erstellung von Schwellwerten für die $O_2^{\bullet-}$- und $CM^{\bullet}$-Konzentration ohne Stratifizierung nach Geschlecht. In Abhängigkeit von Sensitivität und Spezifität wurden exemplarisch Schwellwerte ("Thresholds") für Superoxid und das CM-Radikal bei Vorliegen einer schweren AR aufgeführt (Tab. 3 und 4), bei denen das Geschlecht des Patienten unberücksichtigt bleibt. Die zugrundeliegenden Datensätze entsprechen denjenigen, die hinter den ersten beiden ROC-Diagrammen von Abb. 5 liegen und welche mittels R-Pakets *pROC* auf der Grundlage der bisher erfassten Studiendaten berechnet wurden.

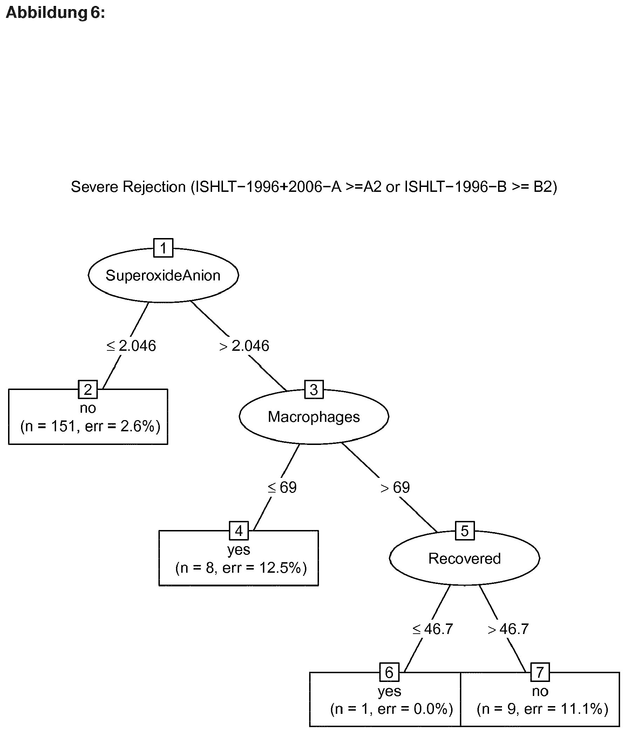

**Beispiel 7:** Für die Erstellung eines Entscheidungsbaums zur Diagnose einer schweren AR (Abb. 6) wurde der C5.0-Algorithmus unter Verwendung folgender Daten eingesetzt: $O_2^{\bullet-}$- und $CM^{\bullet}$-Konzentration, Anteile der Neutrophilen, Makrophagen, Eosinophilen, Lymphozyten, Hämosiderin-Score, Gesamtzellzahl der Leukozyten in der BALF, Recovered [%], $FEV_1$, Alter, Geschlecht sowie die Transplantatüberlebenszeit. Von diesen Daten sah der Algorithmus nur die $O_2^{\bullet-}$-Konzentration, den Anteil an Makrophagen und den zurückgewonnenen Anteil an BALF ("Recovered") als mit akzeptablen Fehlerraten behaftet an.

**Beispiel 8:** In einer nach dem Geschlecht der Patienten getrennten Darstellung zeigen sich sowohl hinsichtlich der $CM^{\bullet}$- und $O_2^{\bullet-}$-Konzentration als auch hinsichtlich des Neutrophilen- und Eosinophilenanteils deutliche Unterschiede (Abb. 7).

**Beispiel 9:** Bei männlichen Ltx-Patienten erscheinen sowohl die $CM^{\bullet}$- als auch die $O_2^{\bullet-}$-Konzentration mit einer AUC von 0,96a.u. als optimale Prädiktoren einer schweren AR (Abb. 7). Bei Frauen liegt die Vorhersagegüte von $CM^{\bullet}$ mit einer AUC von 0,59 a. u. und $O_2^{\bullet-}$ mit einer AUC von 0,72 a.u. deutlich niedriger. Klinisch wichtiger noch verläuft die ROC-Kurve im Anfangsbereich auch weniger steil. Hier erwies sich eine Kombination mehrerer potentieller Prädiktoren sinnvoll, wie sie in Gleichung 2 in Ansatz gebracht wurden.

Die Vorhersagegüte für eine schwere AR bei weiblichen Patienten wird durch die Heuristik besonders im klinisch interessanten Bereich hoher Spezifität deutlich besser, was in Abb. 8 beim Vergleich der Ergebnisse der Heuristik mit den aus der alleinigen Verwendung von $O_2^{\bullet-}$ resultierenden Ergebnissen nachvollziehbar wird. Die Tab. 5 und 6a geben eine in 5 %-Schritten der Spezifität gestufte Auswahl an Schwellwerten wieder, die in vollständiger Form hinter den zugehörigen ROC-Diagrammen (Abb. 7 und 8) liegen und mittels *R*-Pakets *pROC* auf der Grundlage der bisher erfassten Studiendaten berechnet wurden.

**Beispiel 10:** Würde sich bei einer weiblichen Patientin mit dem beschriebenen ESR-Messverfahren einen Wert von 1,2 a.u. für Superoxid ergeben und fänden sich 30% Makrophagen und keine Eosinophilen in der BALF, von der wiederum 80 % zurückgewonnen wurden, so ergäbe sich aus Gleichung 2 ein Score-Wert von 0,0005 a.u./%. Ein Blick in die zugehörige Tab. 6a ergibt dann, dass dieser Wert größer als der dort verzeichnete Schwellwert von 0,000427, aber kleiner als der nachfolgende Wert 0,000519 a.u./% ist. Gibt man sich nun mit einer Spezifität von 85 % zufrieden, dann liegt eine schwere AR vor. Ist die geforderte, z. B. nach ökonomischen Kriterien festgelegte Spezifität aber 90 %, so besteht auf der Grundlage der bisher erfassten Daten eher keine schwere AR.

**Beispiel 11:** Für die Diagnose einer schweren AR bei männlichen Patienten scheint sich Superoxid alleine bisher am besten zu eignen. Würde sich hier beispielsweise ein Messwert für Superoxid ($O_2^{\bullet-}$) von 1,98a.u. ergeben, so läge nach Tab. 6b bei einer vom Anwender gewählten Spezifität von 95 % eine schwere AR vor, nachdem 1,98a.u. größer als der zu einer Spezifität von 95 % zugehörige Schwellwert 1,95a.u. ist. Würde nicht nach Geschlecht unterschieden und wäre der Analyt Superoxid mit dem gleichen Messwert, würde Tabelle 3 (anstatt Tabelle 6b) herangezogen. Bei gleich gewählter Spezifität würde sich ein Schwellwert von 2,12 a.u. ergeben und somit eine schweren AR verneint werden.

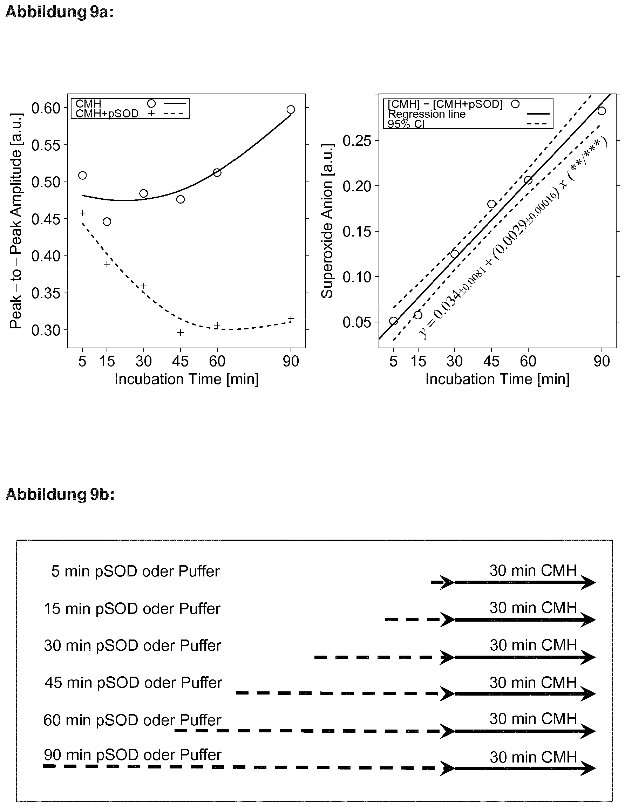

**Beispiel 12:** Abb. 9a und 9b demonstrieren, wenn auch an pulmonal-arteriellen glatten Muskelzellen (*pulmonary arterial smooth muscle cells*, PASMC), dass hinsichtlich einer Verkürzung der Inkubationszeiten Optimierungspotential besteht.

**Beispiel 13:** Zur Erstellung von Referenzbereichen für Makrophagen, Neutrophile, Eosinophile und Lymphozyten bei lungentransplantierten Patienten nach Grad der AR wurde mittels der Funktion *bayesboot* aus dem gleichnamigen *R*-Paket auf Basis von 10000 Stichproben eine parameterfreie Anpassung berechnet und auf dieser Basis die Quantile in Tab. 7 ausgegeben. Unter Verwendung des *R*-Pakets *modeest* wurde außerdem der Modus, also das jeweilige Maximum der Wahrscheinlichkeitsdichte nach der Methode von Parzen bestimmt. Aufgrund der in den ROC-Diagrammen in Bsp. 8 sich abzeichnenden, geschlechtsspezifischen Unterschieden wurden die Referenzbereiche separat für Männern und Frauen berechnet. Das fett gedruckte Quantil 0,5 entspricht dem Median.

## Legenden für Gleichungen, Abbildungen und Tabellen

**Gleichung 1:** Die linksschiefe Verteilung der histopathologischen Diagnosen nach ISHLT (Tab. 2: viele negative und geringgradige, wenige schwere AR-Fälle), verbunden mit der offenbaren Schwierigkeit (Tab. 1), zwischen den nach ISHLT geringgradigen ($A_1$, $B_1$) und den negativen ($A_0$, $B_0$) Fällen zu unterscheiden, erfordert eine Verminderung der Stufenzahl. Deshalb wird hier die Definition einer moderaten oder schweren AR im Unterschied zum Nichtbestehen einer AR in Abhängigkeit der Einstufungen nach ISHLT-A und ISHLT-B vorgenommen. Das Symbol "&" steht für die Bool'sche "und" -Verknüpfung, das Symbol "‖" repräsentiert die "oder"-Verknüpfung, während "$\in$" als "ist ein Element des nachfolgenden Bereichs" zu lesen ist. **Gleichung 2:** Gemäß dieser, nur auf weibliche Patienten anzuwendenden Heuristik werden mit dem Auftreten einer AR positiv korrelierte Messwerte untereinander multipliziert, während durch negativ korrelierte dividiert wird. "Recovered" entspricht dem Verhältnis von zurückgewonnener zu instillierter Spülflüssigkeit in Prozent. Die Addition von 1 zum Prozentsatz der Eosinophilen reflektiert den Umstand, dass Eosinophile in der BALF nur selten auftreten, womit deren Prozentsatz meistens 0 ist und wodurch auch das Produkt meistens 0 würde. Die zusammengefasste Einheit des resultierenden Scores ist a.u./%.

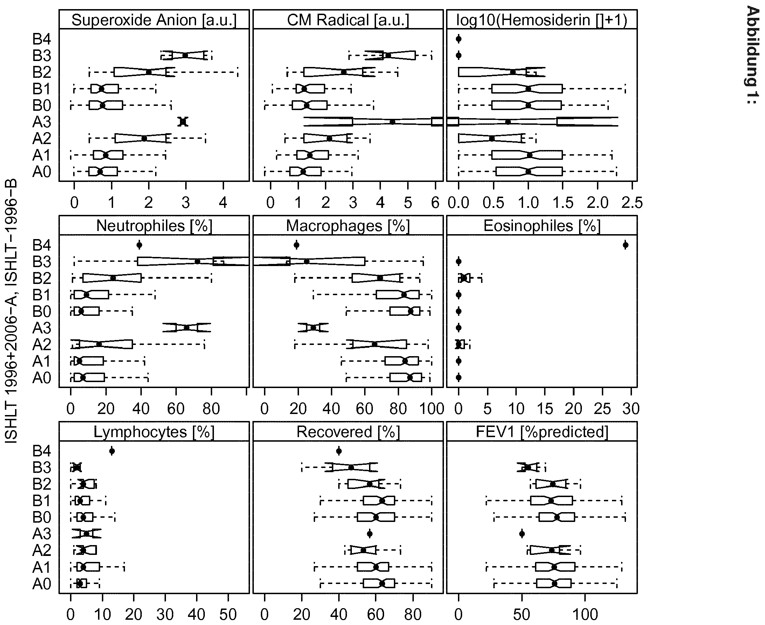

**Abbildung 1:** Detaillierte Boxplots der $O_2^{\bullet-}$- und $CM^{\bullet}$-Konzentration und der BALF-Charakteristika nach ISHLT-A und ISHLT-B sowie klinischer Routinedaten. Es zeichnet sich ein positiver Trend zwischen $O_2^{\bullet-}$- und $CM^{\bullet}$-Konzentration und Neutrophilen mit zunehmenden Abstoßungsgrad ab. Ein negativer Trend ist bei Makrophagen, "Recovered [%]" und Hämosiderin erkennbar. "CM Radical [a.u.]": ESR-Signal nach Abzug des Hintergrundsignals als Maß für die Summe aus $O_2^{\bullet-}$ und $ONOO^-$. "Recovered [%]": zurückgewonnene Lungenspülflüssigkeit, "$FEV_1$ [%predicted]": $FEV_1$ [%Soll]. Zeichenerklärung: Die kleinen offenen Dreiecke markieren Mittelwerte, während die Mediane rund erscheinen. Die Lage des Medians innerhalb des 50 % aller Daten umfassenden Kastens vermittelt einen Eindruck von der Symmetrie der Verteilung der Daten. Die Zahlen darunter geben die Anzahl der Fälle je Abstoßungsgrad an. Die grauen, gestrichelten Ausläufer entsprechen dem 95 % aller Daten umfassenden Konfidenzintervall. Als graue Kreise sind durch den Boxplot-Algorithmus als Ausreißer eingestufte Datenpunkte dargestellt, die sich möglicherweise auf eine fallweise unzutreffende histopathologische Einstufung zurückführen lassen. Falls sich die Einkerbungen in den Boxen zweier Gruppen nicht überlappen und der Mittelwert in der Nähe des Medians zu liegen kommt, besteht wahrscheinlich ein signifikanter Unterschied zwischen diesen Gruppen auch bezüglich ihrer Mittelwerte. Der Hämosiderin-Score wurde zur Verdeutlichung des in den Daten enthaltenen Trends logarithmiert, wobei für "negativ" ein Score-Wert von 0 angenommen wurde. Die Addition von 1 wurde vorgenommen, um den Ausdruck $\log(0) = -\infty$ zu vermeiden. "Rejection class": Abstoßungsklasse "Recovered [%]": zurückgewonnene Lungenspülflüssigkeit, "FEV$_1$[%predicted]": FEV$_1$ [%Soll].

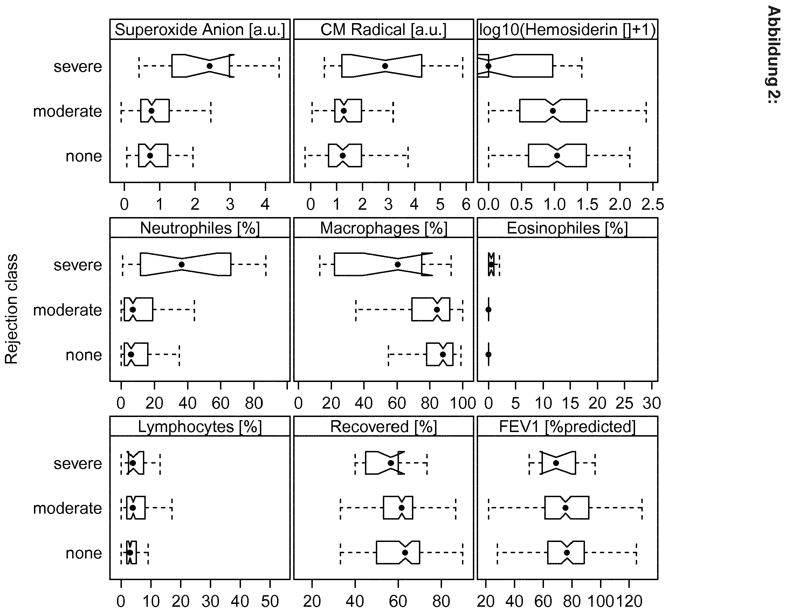

**Abbildung 2:** Boxplots der $O_2^{\bullet-}$- und CM$^\bullet$-Konzentrationen sowie anderer BALF-Charakteristika nach Klassifizierung gemäß Gleichung 1 nebst einer Auswahl klinischer Routinedaten. "CM Radical [a.u.]": ESR-Signal der Proben nach Abzug des Hinter- grundsignals zellfreier Kontrollen als Maß für die Summe aus $O_2^{\bullet-}$ und ONOO$^-$. Zeichenerklärung: siehe Abb. 1.

**Abbildung 3:** Vergleich der $O_2^{\bullet-}$-Konzentrationen bei keiner, einer moderaten oder schweren AR. Links: die $O_2^{\bullet-}$-Konzentration ist bei Vorliegen einer schweren AR im Vergleich zu einer moderaten oder keiner AR hochsignifikant erhöht. Die dünnen Linien zeigen den Standardfehler. Rechts: Statistischer Vergleich zwischen einer schweren AR und keiner bzw. einer moderaten AR. Dicke Linien repräsentieren den Standardfehler der Differenz der Gruppenmittelwerte, dünne Linien das zugehörige 95 %CI ***: $p < 0{,}001$; **: $p < 0{,}01$; * : $p < 0{,}05$; ns : $p \geq 0{,}05$ Die Einteilung in keine (none), eine moderate (moderate) und eine schwere (severe) AR erfolgte nach Glg. 1. Die zugehörigen Fallzahlen sind in Tab. 2 aufgeführt.

**Abbildung 4:** Vergleich der CM$^\bullet$-Konzentrationen bei Einstufung als keine, moderate oder schwere AR nach Glg. 1. Links: Die CM$^\bullet$-Konzentration ist bei Vorliegen einer schweren AR im Vergleich zu einer moderaten oder keiner AR sehr bzw. hoch signifikant erhöht. Rechts: Statistischer Vergleich zwischen einer schweren AR und keiner bzw. einer moderaten AR. Zeichenerklärung: siehe Abb. 3.

**Abbildung 5:** Zu den moderaten und starken Prädiktoren für ein schwere AR zählen die $O_2^{\bullet-}$- und die CM$^\bullet$-Konzentration, Makrophagen und Neutrophile. Eine geringe Vorhersagegüte zeigen dagegen Lymphozyten und FEV$_1$. "CM Radikal [a.u.]": ESR-Signal der Proben nach Abzug des Hintergrundsignals zellfreier Kontrollen als Maß für die Summe aus $O_2^{\bullet-}$ und ONOO-. Die hellgrau hinterlegten Bereiche links im Bild markieren einen hier als klinisch praktikabel angenommenen Spezifitätsbereich zwischen 0,9 und 1. "Recovered [%]": Zurückgewonnener Anteil der BALF. y-Achse: Sensitivität; x-Achse: Spezifität, "AUC": Area Under Curve, Bereichsangabe nach AUC: 95% CI der AUC.

**Abbildung 6:** Beispiel für ein KI-Modells in Form eines Entscheidungsbaums für eine schwere AR auf Basis des C5.0-Algorithmus. Die Superoxid-Konzentration hat die höchste Aussagekraft hinsichtlich der Vorhersage einer schweren AR (1 = Wurzelknoten des Entscheidungsbaums). Bei einer $O_2^{\bullet-}$-Konzentration von $\leq 2.046$ lag in 164 von 182 Fällen keine schwere AR vor (Knoten 2). War die $O_2^{\bullet-}$-Konzentration > 2,046 und der Makrophagenanteil (Knoten 3) in der BALF $\leq 69$ % wurden 8 von 18 Fällen als schwere AR klassifiziert (Knoten 4). Die zurückgewonnene BALF ("Recovered") wurde anhand des C5.0-Algorithmus als dritter, relevanter Parameter für eine Vorhersage einer schweren AR eingeordnet (Knoten 5). err: Anteil der falsch positiv oder falsch negativ klassifizierten Parameter.

**Abbildung 7:** Geschlechtsspezifischer Vergleich der ROC-Kurven/AUC potentieller Prädiktoren einer schweren AR. Die anhand der AUC bemessene Vorhersagegüte verschiedener Prädiktoren einer schweren AR unterscheidet sich teils stark zwischen den Geschlechtern. Bei Männern (schwarze ROC-Kurven) zeigten $O_2^{\bullet-}$ und CM$^\bullet$ eine sehr hohe AUC, wohingegen bei Frauen (graue ROC-Kurven) für CM$^\bullet$ nahezu keine und $O_2^{\bullet-}$ lediglich eine moderate Vorhersagegüte berechnet wurde. "CM Radical [a.u.]": ESR-Signal der Proben nach Abzug des Hintergrundsignals zellfreier Kontrollen als Maß für die Summe aus $O_2^{\bullet-}$ und ONOO$^-$ Die hellgrau hinterlegten Bereiche links im Bild demarkieren einen hier als klinisch praktikabel angenommenen Spezifitätsbereich zwischen 0,9 und 1. "AUC": Area Under Curve, Bereichsangabe nach AUC-Wert: 95% CI.

**Abbildung 8:** ROC-Kurve/AUCs von $O_2^{\bullet-}$ (hellgrau) und heuristische Kombination mehrerer Prädiktoren (schwarz) bei weiblichen Ltx-Patienten. Durch die Kombination der in Glg. 2 angegebenen Prädiktoren konnte bei Frauen die größte Vorhersagegüte hinsichtlich einer schweren AR erzielt werden. Der hellgrau hinterlegte Bereich links im Bild demarkiert einen hier als klinisch praktikabel angenommenen Spezifitätsbereich zwischen 0,9 und 1.

**Abbildung 9a:** $O_2^{\bullet-}$-Konzentration in Abhängigkeit von der Inkubationsdauer von pSOD (n=2). PASMC wurden für 5, 15, 30, 45, 60 oder 90 min mit pSOD inkubiert und anschließend für weitere 30 min mit CMH versetzt (graue Kreuze). Als Kontrollgruppe dienten Zellansätze, die für 5, 15, 30, 45, 60 oder 90 min zunächst nur in KHB und anschließend für 30 min mit CMH inkubiert wurden (schwarze Kreise). Links: Darstellung der CMH bzw. CMH+pSOD Zeitreihen. Die durchgezogenen Linien sind zur Verdeutlichung des exponentiellen Verlaufs eingezeichnete Splines. Rechts: lineare Regression der $O_2^{\bullet-}$-Produktion als Differenz zwischen den ESR-Signalen der allein mit CMH und den zusätzlich mit pSOD inkubierten Proben in Abhängigkeit von der pSOD-Inkubationsdauer der links dargestellten Zeitreihen. Zeichenerklärung: Das graue Band stellt das 95 % CI der Regressionsgeraden dar. (**/***): sehr signifikanter Intercept und hochsignifikante Steigung. Abk.: PASMC - pulmonal-arterielle glatte Muskelzellen, KHB - Krebs-Henseleit-Puffer.

**Abbildung 9b:** pSOD Inkubationsschema zu Abb. 9a.

**Tabelle 1:** Beschreibung siehe Text.

**Tabelle2:** Fallzahlen der Studie, aufgeschlüsselt nach dem nicht-Vorliegen einer AR, einer moderaten und einer schweren AR.

**Tabelle3:** Schwellwerte für $O_2^{\bullet-}$ nach Abzug des Hintergrundsignals zellfreier Kontrollen in willkürlichen Einheiten (a.u.) für das Vorliegen einer schweren AR in Abhängigkeit von Sensitivität und Spezifität, in Sensitivitätsschritten von ca. 5 %, nicht nach Geschlecht getrennt. Der vollständige Datensatz liegt hinter dem ersten ROC-Diagramm in Abb. 5 (dort "Superoxid Anion [a.u.]").

**Tabelle4:** Schwellwerte für das CM-Radikal nach Abzug des Hintergrundsignals zellfreier Kontrollen für das Vorliegen einer schweren AR in willkürlichen Einheiten (a.u.), in Abhängigkeit von Sensitivität und Spezifität und in Sensitivitätsschritten von ca. 5 %, nicht nach Geschlecht getrennt. Der vollständige Datensatz liegt hinter dem zweiten ROC-Diagramm in Abb. 5 (dort "CM Radical [a.u.]").

**Tabelle5:** Schwellwerte in willkürlichen Einheiten für eine schwere Abstoßung bei weiblichen Patienten auf Basis der $O_2^{\bullet-}$-Konzentration (Abb. 7 und 8, graue Kurvenzüge)

**Tabelle 6a:** Schwellwerte in willkürlichen Einheiten für eine schwere Abstoßung bei weiblichen Patienten auf Basis der in Gleichung 2 beschriebenen Heuristik (Abb. 8, schwarzer Kurvenzug).

**Tabelle 6b:** Schwellwerte in willkürlichen Einheiten für eine schwere Abstoßung bei männlichen Patienten auf Basis der in Gleichung 2 beschriebenen Heuristik (Abb. 7, schwarzer Kurvenzug).

**Tabelle7:** Berechnung von Referenzbereichen für Neutrophile, Makrophagen, Lymphozyten und Eosinophile nach Schweregrad der AR und Geschlecht der Patienten mittels der Funktion *bayesboot* aus dem gleichnamigen *R*-Paket. Die Einteilung in keine, eine moderate oder eine schwere AR erfolgte nach Gleichung 1, wobei die Gruppe der Patienten, bei denen keine Abstoßung vorlag, zusätzlich auf negative CRP-Werte eingeengt wurde, um damit die Wahrscheinlichkeit eines gleichzeitigen Vorliegens einer Infektion weiter zu verringern. Der Median entspricht dem 50% Quantil (0.5). Der Modus gibt das Maximum der Wahrscheinlichkeitsdichte an. Abk.: AR - akute Abstoßung; CRP - C-reaktives Protein.

**Patentansprüche**

1.  Verfahren zur Bestimmung einer akuten Transplantatabstoßung in einer biologischen Probe eines Patienten **dadurch gekennzeichnet, dass** folgende Schritte durchgeführt werden:

    i) Messung der reaktiven Sauerstoffspezies (ROS) und/oder der reaktiven Stickstoffspezies (RNS) in der biologischen Probe, wobei der Analyt der Messung Superoxid ($O_2^{\bullet-}$) und/oder Peroxinitrit ($ONOO^-$) ist
    ii) Vergleich des Messwerts der reaktiven Spezies aus Schritt i) mit dem zugehörigen Schwellwert bei einer zu wählenden Sensitivität oder Spezifität
    iii) Entscheidung für das Vorliegen einer akuten Transplantatabstoßung, wenn der Messwert über dem zugehörigen Schwellwert liegt

2.  Verfahren gemäß Anspruch 1 **dadurch gekennzeichnet, dass** in Schritt i) weitere radikalische bzw. hochreaktive Verbindungen wie $^{\bullet}OH$, $LOOH$, $^{\bullet}NO$, $NO_2^-$ oder $H_2O_2$, aber auch die radikalischen Formen der Ascorbinsäure ($Asc^{\bullet}$) und des $\alpha$-Tocopherols ($Toc^{\bullet}$) gemessen werden.

3.  Verfahren nach einem der vorherigen Ansprüche 1 und 2 **dadurch gekennzeichnet, dass** die Messung aus Schritt i) mittels Elektronen-Spin-Resonanz-Spektroskopie erfolgt.

4.  Verfahren nach einem der vorherigen Ansprüche 1 und 2 **dadurch gekennzeichnet, dass** die Messung aus Schritt i) mittels Fluoreszenz- oder Lumineszenz-basierten Verfahren, mittels Redox-Sensoren oder mittels HPLC, GC/MS, LC/MS oder der spektrophotometrischen Messung der Cytochrom-c Reduktion erfolgt.

5.  Verfahren nach einem der vorherigen Ansprüche **dadurch gekennzeichnet, dass** die biologische Probe eine gasförmige, flüssige oder feste Probe ist.

6.  Verfahren nach einem der vorherigen Ansprüche **dadurch gekennzeichnet, dass** die biologische Probe eine flüssige Probe ist, umfassend Gallenflüssigkeit, Pankreassekret, Liquor cerebrospinalis, Tränenflüssigkeit, Ascites, Blut, Gelenkflüssigkeit, Lymphflüssigkeit, Urin oder eine durch Spülung verdünnte Körperflüssigkeit wie ELF, Pleuraflüssigkeit, Perikardflüssigkeit, Peritonealflüssigkeit ist oder eine verflüssigte feste Probe ist.

7.  Verfahren nach einem der vorherigen Ansprüche 1-5 **dadurch gekennzeichnet, dass** die biologische Probe eine feste Probe ist, umfassend ein Bioptat oder eine eingefrorene flüssige Probe.

8. Verfahren nach einem der vorherigen Ansprüche 1-5 **dadurch gekennzeichnet, dass** die biologische Probe eine gasförmige Probe ist, umfassend Atemluft eines Patienten oder eine durch Katheter aus Körperhöhlen entnommene gasförmige Probe.

9. Verfahren nach einem der vorherigen Ansprüche **dadurch gekennzeichnet, dass** nach Schritt iii) ein zusätzlicher Schritt iv) durchgeführt wird, in dem die Makrophagenkonzentration in der biologischen Probe ermittelt wird.

10. Verfahren nach Anspruch 9 **dadurch gekennzeichnet, dass** nach Schritt iv) ein zusätzlicher Schritt v) durchgeführt wird, in dem ein Flüssigkeitsvolumen als Recovered [%]-Wert ermittelt wird, wobei die Flüssigkeit über eine BAL gewonnen wird und wobei das instillierte als auch das nach Absaugen zurückgewonnene Volumen (BALF) ermittelt wird.

11. Verfahren nach einem der vorherigen Ansprüche 1 bis 8 **dadurch gekennzeichnet, dass** in Schritt iv) zusätzlich auch die Eosinophilenkonzentration in der biologischen Probe ermittelt wird und wobei ein Score-Wert nach Gleichung 2 ermittelt wird:

$$Score = Superoxid / Makrophagen \times (Eosinophile +1) / Recovered$$

12. Verfahren nach einem der vorherigen Ansprüche 1 bis 8 **dadurch gekennzeichnet, dass** in Schritt ii) die Schwellwerte unter Berücksichtigung des Geschlechts des Patienten ausgewählt werden.

13. Verwendung eines Verfahrens gemäß der Ansprüche 1-12 zur Therapieüberwachung und/oder Dosisanpassung von Immunsuppressiva zur Therapie der akuten Transplantatabstoßung.

14. Vorrichtung zur Durchführung eines Verfahrens gemäß der Ansprüche 1-12 umfassend

    - eine Messeinheit zur Eingabe der biologischen Probe und zur Erfassung von ROS und/oder RNS in der biologischen Probe durch Messung und
    - eine Auswerteinheit, die mit der Messeinheit verbunden ist, in der die Messwerte aus der Messeinheit mit jeweils zugehörigen Schwellwerten abgeglichen werden und die Auswertung erfolgt, ob eine akute Transplantatabstoßung vorliegt oder nicht.

15. Vorrichtung gemäß Anspruch 14 **dadurch gekennzeichnet, dass** die Messeinheit und die Auswerteinheit automatisiert zusammenwirken und die Auswerteeinheit eine Einheit des maschinellem Lernens (KI) umfasst.

**Abbildung 1:**

Abbildung 2:

**Abbildung 3:**

**Abbildung 4:**

Abbildung 5:

**Abbildung 6:**

Severe Rejection (ISHLT−1996+2006−A >=A2 or ISHLT−1996−B >= B2)

**Abbildung 7:**

**Abbildung 8:**

**Abbildung 9a:**

**Abbildung 9b:**

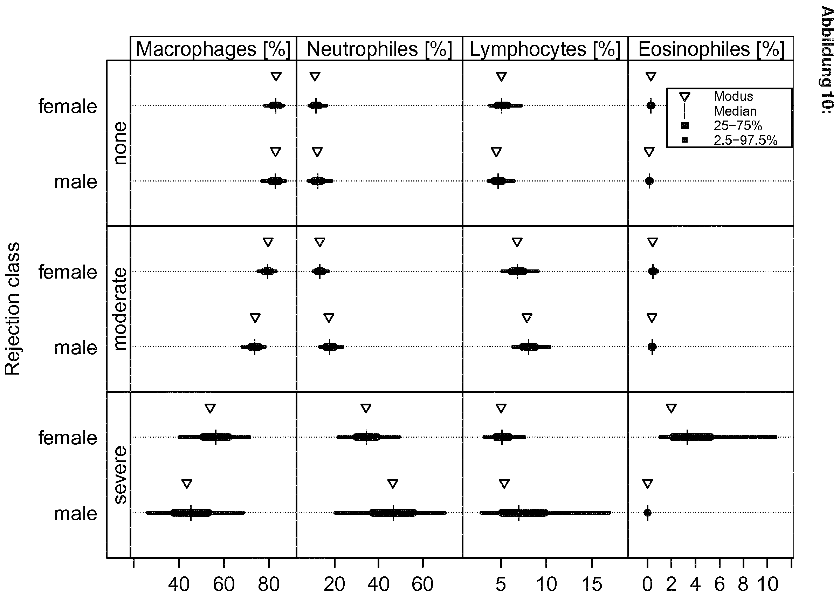

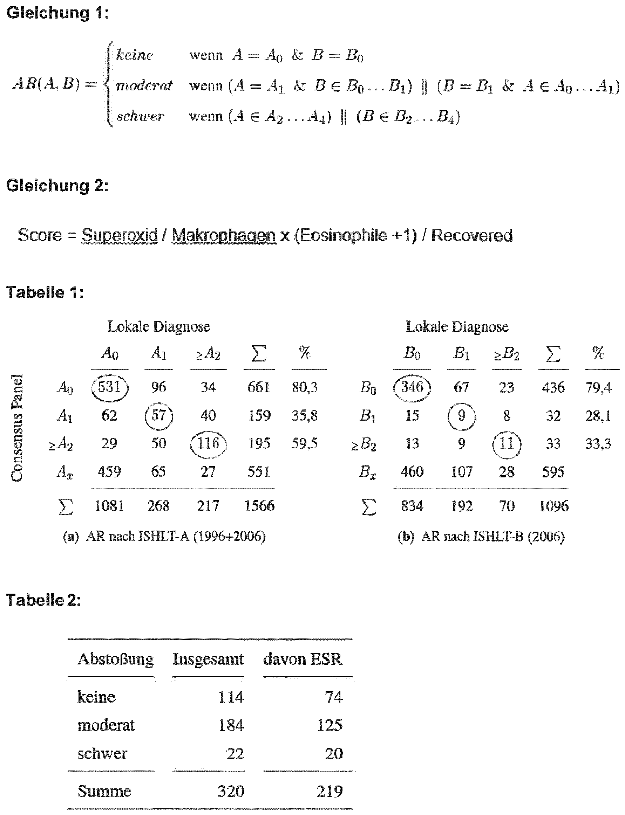

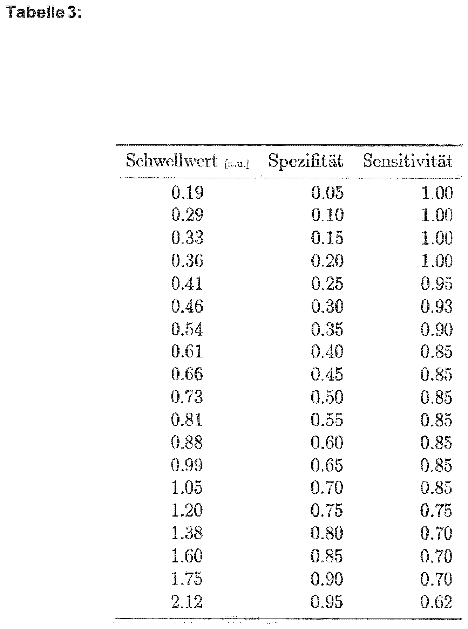

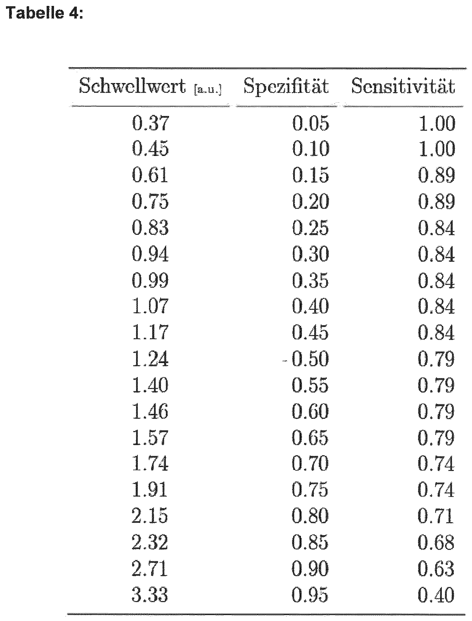

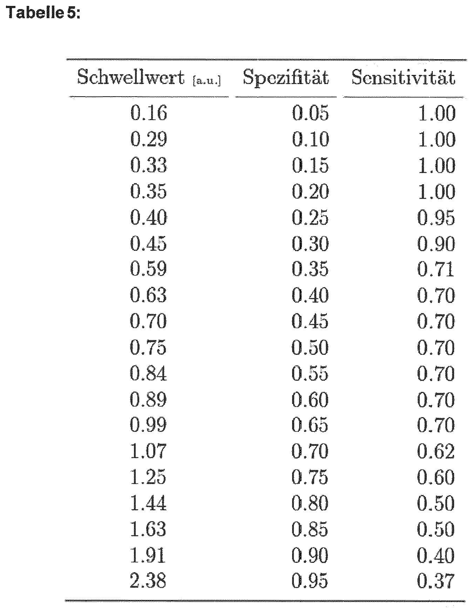

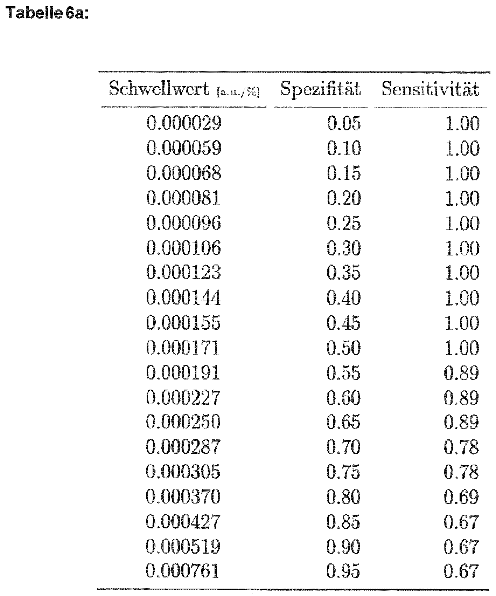

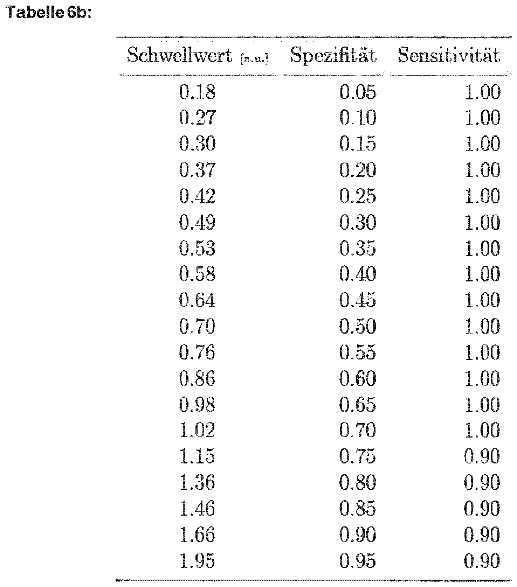

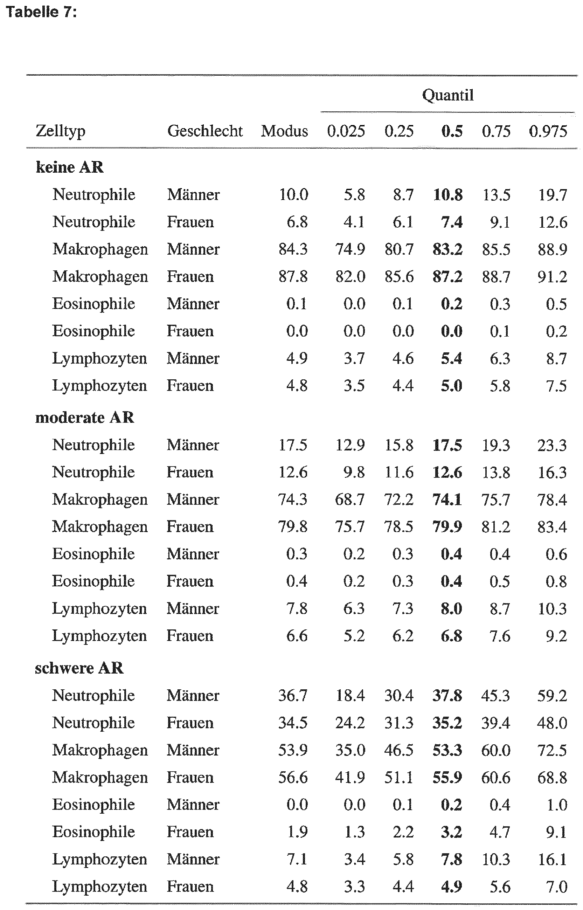

**Abbildung 10:**

**Gleichung 1:**

$$AR(A,B) = \begin{cases} keine & \text{wenn } A = A_0 \ \& \ B = B_0 \\ moderat & \text{wenn } (A = A_1 \ \& \ B \in B_0 \ldots B_1) \ \| \ (B = B_1 \ \& \ A \in A_0 \ldots A_1) \\ schwer & \text{wenn } (A \in A_2 \ldots A_4) \ \| \ (B \in B_2 \ldots B_4) \end{cases}$$

**Gleichung 2:**

Score = Superoxid / Makrophagen x (Eosinophile +1) / Recovered

**Tabelle 1:**

| | | Lokale Diagnose | | | | | | Lokale Diagnose | | | |
|---|---|---|---|---|---|---|---|---|---|---|---|
| | | $A_0$ | $A_1$ | $\geq A_2$ | $\sum$ | % | | $B_0$ | $B_1$ | $\geq B_2$ | $\sum$ | % |
| Consensus Panel | $A_0$ | (531) | 96 | 34 | 661 | 80,3 | $B_0$ | (346) | 67 | 23 | 436 | 79,4 |
| | $A_1$ | 62 | (57) | 40 | 159 | 35,8 | $B_1$ | 15 | (9) | 8 | 32 | 28,1 |
| | $\geq A_2$ | 29 | 50 | (116) | 195 | 59,5 | $\geq B_2$ | 13 | 9 | (11) | 33 | 33,3 |
| | $A_x$ | 459 | 65 | 27 | 551 | | $B_x$ | 460 | 107 | 28 | 595 | |
| | $\sum$ | 1081 | 268 | 217 | 1566 | | $\sum$ | 834 | 192 | 70 | 1096 | |

(a) AR nach ISHLT-A (1996+2006)   (b) AR nach ISHLT-B (2006)

**Tabelle 2:**

| Abstoßung | Insgesamt | davon ESR |
|---|---|---|
| keine | 114 | 74 |
| moderat | 184 | 125 |
| schwer | 22 | 20 |
| Summe | 320 | 219 |

Tabelle 3:

| Schwellwert [a.u.] | Spezifität | Sensitivität |
|---|---|---|
| 0.19 | 0.05 | 1.00 |
| 0.29 | 0.10 | 1.00 |
| 0.33 | 0.15 | 1.00 |
| 0.36 | 0.20 | 1.00 |
| 0.41 | 0.25 | 0.95 |
| 0.46 | 0.30 | 0.93 |
| 0.54 | 0.35 | 0.90 |
| 0.61 | 0.40 | 0.85 |
| 0.66 | 0.45 | 0.85 |
| 0.73 | 0.50 | 0.85 |
| 0.81 | 0.55 | 0.85 |
| 0.88 | 0.60 | 0.85 |
| 0.99 | 0.65 | 0.85 |
| 1.05 | 0.70 | 0.85 |
| 1.20 | 0.75 | 0.75 |
| 1.38 | 0.80 | 0.70 |
| 1.60 | 0.85 | 0.70 |
| 1.75 | 0.90 | 0.70 |
| 2.12 | 0.95 | 0.62 |

**Tabelle 4:**

| Schwellwert [a.u.] | Spezifität | Sensitivität |
|---|---|---|
| 0.37 | 0.05 | 1.00 |
| 0.45 | 0.10 | 1.00 |
| 0.61 | 0.15 | 0.89 |
| 0.75 | 0.20 | 0.89 |
| 0.83 | 0.25 | 0.84 |
| 0.94 | 0.30 | 0.84 |
| 0.99 | 0.35 | 0.84 |
| 1.07 | 0.40 | 0.84 |
| 1.17 | 0.45 | 0.84 |
| 1.24 | 0.50 | 0.79 |
| 1.40 | 0.55 | 0.79 |
| 1.46 | 0.60 | 0.79 |
| 1.57 | 0.65 | 0.79 |
| 1.74 | 0.70 | 0.74 |
| 1.91 | 0.75 | 0.74 |
| 2.15 | 0.80 | 0.71 |
| 2.32 | 0.85 | 0.68 |
| 2.71 | 0.90 | 0.63 |
| 3.33 | 0.95 | 0.40 |

**Tabelle 5:**

| Schwellwert [a.u.] | Spezifität | Sensitivität |
|:---:|:---:|:---:|
| 0.16 | 0.05 | 1.00 |
| 0.29 | 0.10 | 1.00 |
| 0.33 | 0.15 | 1.00 |
| 0.35 | 0.20 | 1.00 |
| 0.40 | 0.25 | 0.95 |
| 0.45 | 0.30 | 0.90 |
| 0.59 | 0.35 | 0.71 |
| 0.63 | 0.40 | 0.70 |
| 0.70 | 0.45 | 0.70 |
| 0.75 | 0.50 | 0.70 |
| 0.84 | 0.55 | 0.70 |
| 0.89 | 0.60 | 0.70 |
| 0.99 | 0.65 | 0.70 |
| 1.07 | 0.70 | 0.62 |
| 1.25 | 0.75 | 0.60 |
| 1.44 | 0.80 | 0.50 |
| 1.63 | 0.85 | 0.50 |
| 1.91 | 0.90 | 0.40 |
| 2.38 | 0.95 | 0.37 |

Tabelle 6a:

| Schwellwert [a.u./%] | Spezifität | Sensitivität |
|---|---|---|
| 0.000029 | 0.05 | 1.00 |
| 0.000059 | 0.10 | 1.00 |
| 0.000068 | 0.15 | 1.00 |
| 0.000081 | 0.20 | 1.00 |
| 0.000096 | 0.25 | 1.00 |
| 0.000106 | 0.30 | 1.00 |
| 0.000123 | 0.35 | 1.00 |
| 0.000144 | 0.40 | 1.00 |
| 0.000155 | 0.45 | 1.00 |
| 0.000171 | 0.50 | 1.00 |
| 0.000191 | 0.55 | 0.89 |
| 0.000227 | 0.60 | 0.89 |
| 0.000250 | 0.65 | 0.89 |
| 0.000287 | 0.70 | 0.78 |
| 0.000305 | 0.75 | 0.78 |
| 0.000370 | 0.80 | 0.69 |
| 0.000427 | 0.85 | 0.67 |
| 0.000519 | 0.90 | 0.67 |
| 0.000761 | 0.95 | 0.67 |

**Tabelle 6b:**

| Schwellwert [a.u.] | Spezifität | Sensitivität |
| --- | --- | --- |
| 0.18 | 0.05 | 1.00 |
| 0.27 | 0.10 | 1.00 |
| 0.30 | 0.15 | 1.00 |
| 0.37 | 0.20 | 1.00 |
| 0.42 | 0.25 | 1.00 |
| 0.49 | 0.30 | 1.00 |
| 0.53 | 0.35 | 1.00 |
| 0.58 | 0.40 | 1.00 |
| 0.64 | 0.45 | 1.00 |
| 0.70 | 0.50 | 1.00 |
| 0.76 | 0.55 | 1.00 |
| 0.86 | 0.60 | 1.00 |
| 0.98 | 0.65 | 1.00 |
| 1.02 | 0.70 | 1.00 |
| 1.15 | 0.75 | 0.90 |
| 1.36 | 0.80 | 0.90 |
| 1.46 | 0.85 | 0.90 |
| 1.66 | 0.90 | 0.90 |
| 1.95 | 0.95 | 0.90 |

**Tabelle 7:**

| Zelltyp | Geschlecht | Modus | Quantil | | | | |
|---|---|---|---|---|---|---|---|
| | | | 0.025 | 0.25 | **0.5** | 0.75 | 0.975 |
| **keine AR** | | | | | | | |
| Neutrophile | Männer | 10.0 | 5.8 | 8.7 | **10.8** | 13.5 | 19.7 |
| Neutrophile | Frauen | 6.8 | 4.1 | 6.1 | **7.4** | 9.1 | 12.6 |
| Makrophagen | Männer | 84.3 | 74.9 | 80.7 | **83.2** | 85.5 | 88.9 |
| Makrophagen | Frauen | 87.8 | 82.0 | 85.6 | **87.2** | 88.7 | 91.2 |
| Eosinophile | Männer | 0.1 | 0.0 | 0.1 | **0.2** | 0.3 | 0.5 |
| Eosinophile | Frauen | 0.0 | 0.0 | 0.0 | **0.0** | 0.1 | 0.2 |
| Lymphozyten | Männer | 4.9 | 3.7 | 4.6 | **5.4** | 6.3 | 8.7 |
| Lymphozyten | Frauen | 4.8 | 3.5 | 4.4 | **5.0** | 5.8 | 7.5 |
| **moderate AR** | | | | | | | |
| Neutrophile | Männer | 17.5 | 12.9 | 15.8 | **17.5** | 19.3 | 23.3 |
| Neutrophile | Frauen | 12.6 | 9.8 | 11.6 | **12.6** | 13.8 | 16.3 |
| Makrophagen | Männer | 74.3 | 68.7 | 72.2 | **74.1** | 75.7 | 78.4 |
| Makrophagen | Frauen | 79.8 | 75.7 | 78.5 | **79.9** | 81.2 | 83.4 |
| Eosinophile | Männer | 0.3 | 0.2 | 0.3 | **0.4** | 0.4 | 0.6 |
| Eosinophile | Frauen | 0.4 | 0.2 | 0.3 | **0.4** | 0.5 | 0.8 |
| Lymphozyten | Männer | 7.8 | 6.3 | 7.3 | **8.0** | 8.7 | 10.3 |
| Lymphozyten | Frauen | 6.6 | 5.2 | 6.2 | **6.8** | 7.6 | 9.2 |
| **schwere AR** | | | | | | | |
| Neutrophile | Männer | 36.7 | 18.4 | 30.4 | **37.8** | 45.3 | 59.2 |
| Neutrophile | Frauen | 34.5 | 24.2 | 31.3 | **35.2** | 39.4 | 48.0 |
| Makrophagen | Männer | 53.9 | 35.0 | 46.5 | **53.3** | 60.0 | 72.5 |
| Makrophagen | Frauen | 56.6 | 41.9 | 51.1 | **55.9** | 60.6 | 68.8 |
| Eosinophile | Männer | 0.0 | 0.0 | 0.1 | **0.2** | 0.4 | 1.0 |
| Eosinophile | Frauen | 1.9 | 1.3 | 2.2 | **3.2** | 4.7 | 9.1 |
| Lymphozyten | Männer | 7.1 | 3.4 | 5.8 | **7.8** | 10.3 | 16.1 |
| Lymphozyten | Frauen | 4.8 | 3.3 | 4.4 | **4.9** | 5.6 | 7.0 |

Europäisches
Patentamt

European
Patent Office

Office européen
des brevets

**EUROPÄISCHER RECHERCHENBERICHT**

Nummer der Anmeldung

EP 18 21 4270

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| T | Susan Scheibe: "Detektion reaktiver Sauerstoffspezies im kardiopulmonalen System mittels Electron Spin Resonance (ESR)-Spektroskopie", , 20. Dezember 2018 (2018-12-20), XP055578806, Gefunden im Internet: URL:http://geb.uni-giessen.de/geb/volltext e/2018/13933/pdf/ScheibeSusan_2018_06_21.p df [gefunden am 2019-04-08] ----- | | INV. G01N33/84 |
| X | HUISMAN A ET AL: "Anti-inflammatory effects of tetrahydrobiopterin on early rejection in renal allografts: modulation of inducible nitric oxide synthase", THE FASEB JOURNAL, FEDERATION OF AMERICAN SOCIETIES FOR EXPERIMENTAL BIOLOGY, US, Bd. 16, Nr. 9, 1. Juli 2002 (2002-07-01), Seiten 1135-1137, XP003002130, ISSN: 0892-6638 | 14,15 | |
| Y | * Abstrakt, Abbildung 1 * ----- | 1-13 | RECHERCHIERTE SACHGEBIETE (IPC)   G01N |
| X | Galen Pieper: "Protective Mechanisms of a Metalloporphyrinic Peroxynitrite Decomposition Catalyst, WW85, in Rat Cardiac Transplants", , 1. Juli 2005 (2005-07-01), XP055579524, Gefunden im Internet: URL:http://jpet.aspetjournals.org/content/ jpet/314/1/53.full.pdf [gefunden am 2019-04-10] * Zusammenfassung * ----- -/-- | 14,15 | |

Der vorliegende Recherchenbericht wurde für alle Patentansprüche erstellt

| Recherchenort | Abschlußdatum der Recherche | Prüfer |
|---|---|---|
| München | 2. Mai 2019 | Behrens, Ralf |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
........................................................
& : Mitglied der gleichen Patentfamilie, übereinstimmendes
Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 18 21 4270

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | AKIZUKI E ET AL: "Role of nitric oxide and superoxide in acute cardiac allograft rejection in rats", PROCEEDINGS OF THE SOCIETY FOR EXPERIMENTAL BIOLOGY AND MEDICINE, SAGE PUBLICATIONS LTD, GB, Bd. 225, Nr. 2, 31. Oktober 2000 (2000-10-31), Seiten 151-159, XP009512512, ISSN: 0037-9727, DOI: 10.1046/J.1525-1373.2000.22519.X * Zusammenfassung * ----- | 14,15 | |
| X | M K Al-Ali ET AL: "Topical Review Nitric oxide and the respiratory system in health and disease", RESPIRATORY MEDICINE, 1. Januar 1998 (1998-01-01), Seiten 701-715, XP055578582, Gefunden im Internet: URL:www. * Abbildung 1 * ----- | 14,15 | |
| X | SHIRAISHI ET AL: "Inhibition of inducible nitric oxide synthase ameliorates rat lung allograft rejection", JOURNAL OF THORACIC AND CARDIOVASCULAR SURG, MOSBY-YEAR BOOK, INC., ST. LOUIS, MO, US, Bd. 110, Nr. 5, 1. November 1995 (1995-11-01), Seiten 1449-1460, XP005144129, ISSN: 0022-5223, DOI: 10.1016/S0022-5223(95)70068-4 | 14,15 | RECHERCHIERTE SACHGEBIETE (IPC) |
| Y | * Zusammenfassung * ----- | 1-13 | |

Der vorliegende Recherchenbericht wurde für alle Patentansprüche erstellt

| Recherchenort | Abschlußdatum der Recherche | Prüfer |
|---|---|---|
| München | 2. Mai 2019 | Behrens, Ralf |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)